(19) Europäisches Patentamt
European Patent Office
Office européen des brevets

(11) EP 1 592 675 B1

(12) EUROPEAN PATENT SPECIFICATION

(45) Date of publication and mention of the grant of the patent:
**08.10.2008 Bulletin 2008/41**

(21) Application number: **04707239.2**

(22) Date of filing: **02.02.2004**

(51) Int Cl.:
***C07D 251/68*** (2006.01) ***D06L 3/12*** (2006.01)

(86) International application number:
**PCT/EP2004/050064**

(87) International publication number:
**WO 2004/069790 (19.08.2004 Gazette 2004/34)**

# (54) CRYSTALLINE MODIFICATIONS OF TRIAZINYLAMINOSTILBENES

KRISTALLMODIFIKATIONEN VON TRIAZINYLAMINOSTILBENEN

MODIFICATION CRISTALLINES DES TRIAZINYLAMINOSTILBENES

(84) Designated Contracting States:
**AT BE BG CH CY CZ DE DK EE ES FI FR GB GR HU IE IT LI LU MC NL PT RO SE SI SK TR**

(30) Priority: **10.02.2003 EP 03405072**

(43) Date of publication of application:
**09.11.2005 Bulletin 2005/45**

(73) Proprietor: **Ciba Holding Inc.**
**4057 Basel (CH)**

(72) Inventors:
- **KASCHIG, Jürgen**
**79104 Freiburg (DE)**
- **ZELGER, Josef**
**CH-4125 Riehen (CH)**
- **SCHRÖDER, Serge**
**F-68300 Rosenau (FR)**
- **HOCHBERG, Robert**
**79249 Merzhausen (DE)**
- **BECHERER, Oliver**
**79739 Schwörstadt (DE)**
- **MEIER, Dieter**
**79591 Eimeldingen (DE)**

(56) References cited:
**DD-A- 145 017**
**GB-A- 1 093 507**
**US-A- 3 951 960**
**US-A- 4 005 026**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention relates to a novel polymorph crystal form of the compound of formula (1)

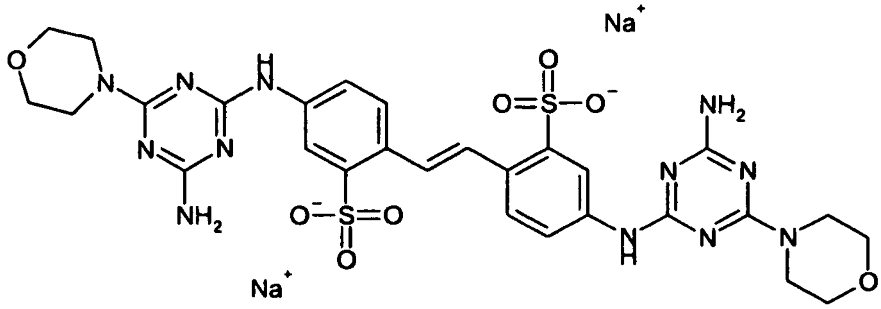

(1)

,

a process for its preparation and the use thereof.

The compound of formula (1) is known as an amorphous modification only.

WO 2003/070870 describes detergents compositions containing a fluorescent whitening agent and a peroxide, a peroxide activator and/or a bleaching catalyst. One of the disclosed fluorescent whitening agents is 4,4'-bis(4-amino-6-morpholino-s-triazin-2-ylamino)-2,2'-stilbenedisulfonic acid, sodium salt, the preparation of which is described in example 3.

DD 145 017 describes crystalline forms of 4,4'-bis(4-anilino-6-morpholino-s-triazin-2-yl-amino)-2,2'-stilbenedisulfonic acid, sodium salt and 4,4'-bis(4,6-dianilino-s-triazin-2-ylamino)-2,2'-stilbenedisulfonic acid, sodium salt, which are both suitable as optical brighteners for use in detergents.

US 4,005,026 and US 3'951'960 describe crystalline needle forms of the fluorescent compounds 4,4'-bis(4-anilino-6-morpholino-s-triazin-2-ylamino)-2,2'-stilbenedisulfonic acid, sodium salt and 4,4'-bis(4,6-dianilino-s-triazin-2-ylamino)-2,2'-stilbenedisulfonic acid, sodium salt useful as optical brightening agents, particularly when incorporated into detergent compositions.

GB 1,093,507 describes a crystalline form of 4,4'-bis(4-anilino-6-morpholino-s-triazin-2-ylamino)-2,2'-stilbenedisulfonic acid.

The ability of a substance to exist in more than one crystal form is defined as polymorphism and these different crystal forms are named "polymorph modifications" or "polymorphs". In general, polymorphism is affected by the ability of a molecule of a substance to change its conformation or to form different intermolecular or intra-molecular interactions, particularly hydrogen bonds, which is reflected in different atom arrangements in the crystal lattices of different polymorphs. Polymorphism is found in several organic compounds.

The different polymorphs of a substance possess different energies of the crystal lattice and, thus, in solid state they show different physical properties.

A useful tool for the differentiation of crystal modification is the X-ray diffraction, such as for example the powder X-ray diffraction.

Due to the fact that the crystal form and therefore also the powder X-ray diffraction pattern is among others dependent on the degree of dryness, the content of water is specified for each spectrum.

The content of water is measured by common known analytical methods. In the present application, the content of water has been measured by the Karl Fischer Titration.

All X-ray powder spectra data have been obtained by X-ray diffraction on a powder sample of the new crystal modification in the instrument X'Pert powder diffractometer PANalytical inside a closed Anton Paar measuring stage using $CuK_\alpha$ radiation. The followong conditions have been set:

- tube power 40kV/40mA
- scan range 1 - 50° (20)
- Step size 0.02° (2θ
- time per step 2s
- room temperature.

The present invention relates to a crystal modification of the compound of formula (1)

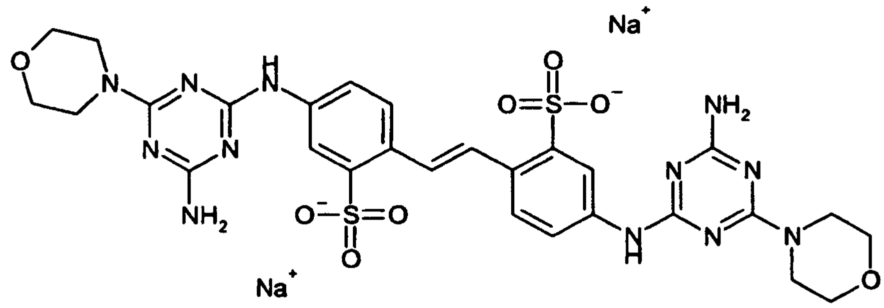

(1) ,

characterized by
peaks at about 21.8, 11.0 and 7.3 d-spacing units in its powder X-ray diffraction pattern at a content of water > 0%.

Specifically, the new crystal modification of the compound of formula (1) with a content of water ≥ 19% is characterized by peaks at about 21.8, 11.6, 11.0, 9.2, 7.3, 5.82, 4.37, 4.05, 3.96, 3.88, 3.36, 3.1, 3.04, 2.95, 2.8, 2.56 and 2.32 d-spacing units in its powder X-ray diffraction pattern.

More specifically, the new crystal modification of the compound of formula (1) with content of water ≥ 19% is characterized by peaks at about 21.8, 11.8, 11.6, 11.0, 9.2, 7.3, 5.82, 4.48, 4.37. 4.05, 3.96, 3.88, 3.4, 3.36, 3.18, 3.1. 3.04, 2.95, 2.91, 2.86, 2.80, 2.78, 2.56, 2.41, 2.36 and 2.32 d-spacing units in its powder X-ray diffraction pattern.

Table 1 shows the characteristic spacing between the lattice planes designated by d and expresses in Angström units [A] and their corresponding characteristic relative intensity (very weak, weak, medium, strong or very strong) of the new crystal modification of the compound of formula (1) with content of water ≥19% obtained by Example 1. Instead of d it is also common to use 2θ. The following formula shows the relation between d and 2θ:

$$d\,(\text{Å}) = \frac{1.54060\ \text{Å}}{2\sin(2\theta/2)}$$

Table 1

| d-spacing (Å) | Relative intensity | d-spacing (Å) | Relative intensity | d-spacing (Å) | Relative intensity |
|---|---|---|---|---|---|
| 21.8 | vw | 4.75 | w | 3.36 | s |
| 11.8 | m | 4.68 | w | 3.29 | vw |
| 11.6 | s | 4.48 | m | 3.18 | vw |
| 11.0 | w | 4.37 | s | 3.1 | w |
| 9.2 | s | 4.21 | vw | 3.04 | w |
| 7.3 | vw | 4.05 | m | 2.95 | m |
| 7.2 | vw | 3.96 | m | 2.91 | vw |
| 7.0 | w | 3.88 | vs | 2.86 | vw |
| 6.1 | w | 3.83 | vw | 2.8 | w |
| 5.82 | vs | 3.73 | vw | 2.78 | w |
| 5.5 | vw | 3.64 | vw | 2.56 | m |
| 5.39 | vw | 3.51 | vw | 2.41 | vw |
| 5.1 | w | 3.45 | vw | 2.36 | vw |
| 5.02 | w | 3.4 | vw | 2.32 | w |

Fig. 1. shows the powder X-ray diffraction pattern of the new crystal modification of the compound of formula (1) with a content of water ≥ 19% obtained by Example 1.

The invention relates also to a new crystal modification of the compound of formula (1) with a content of water < 19% and ≥ 17%, characterized by peaks at about 21.8, 11.7, 11.0, 9.2, 7.30, 5.78 and 3.87 d-spacing units in its powder X-ray diffraction pattern.

Specifically, the new crystal modification of the compound of formula (1) with a content of water < 19% and ≥ 17%, is characterized by peaks at about 21.8, 11.7, 11.5, 11.0, 7.3, 5.78, 4.95, 4.04, 3.87 and 3.71 d-spacing units in its powder X-ray diffraction pattern.

More specifically, the new crystal modification of the compound of formula (1) with a content of water < 19% and ≥ 17%, is characterized by peaks at about 21.8, 11.7, 11.5, 11.0, 9.2, 7.3, 7.2, 7.0, 5.78, 5.56, 4.95, 4.75, 4.6, 4.49, 4.31, 4.15, 4.04, 3.94, 3.87, 3.71, 3.46, 3.36, 3.16, 2.96 and 2.56 d-spacing units in its powder X-ray diffraction pattern.

Table 2 shows the characteristic spacing between the lattice planes designated by d and expresses in Angstöm units [A] and their corresponding characteristic relative intensisty (every weak, weak, medium, strong or very strong) of the new crystal modification of the compound of formula (1) with a content of water < 19% and ≥ 17% obtained by Example 2.

Table 2

| d-spacing (Å) | Relative intensity | d-spacing (Å) | Relative intensity | d-spacing (Å) | Relative intensity |
|---|---|---|---|---|---|
| 21.8 | w | 5.56 | m | 3.94 | w |
| 11.7 | s | 5.28 | w | 3.87 | s |
| 11.5 | m | 4.95 | m | 3.71 | m |
| 11.0 | m | 4.75 | m | 3.46 | w |
| 9.2 | s | 4.6 | w | 3.36 | w |
| 7.3 | m | 4.49 | m | 3.16 | w |
| 7.2 | m | 4.41 | w | 2.96 | w |
| 7.0 | m | 4.31 | w | 2.82 | w |
| 6.0 | w | 4.15 | w | 2.56 | w |
| 5.78 | vs | 4.04 | m | | |

Fig. 2. shows the powder X-ray diffraction pattern of the new crystal modification of the compound of formula (1) with a content of water < 19% and≥ 17% obtained by Example 2.

The invention relates also to a new crystal modification of the compound of formula (1) with a content of water < 17% and ≥ 13%, characterized by peaks at about about 21.8, 11.7, 11.0, 7.30, 5.77, 5.51, 4.93 and 3.65 d-spacing units in its powder X-ray diffraction pattern.

Specifically, the new crystal modification of the compound of formula (1) with a content of water < 17% and ≥ 13%, is characterized by peaks at about about 21.8, 11.6, 11.0, 9.2, 7.3, 7.2. 5.77, 5.51, 4.93, 4.49, 3.86 and 3.65 d-spacing units in its powder X-ray diffraction pattern.

Table 3 shows the characteristic spacing between the lattice planes designated by d and expresses in Angstöm units [A] and their corresponding characteristic relative intensisty (very weak, weak, medium, strong or very strong) of the new crystal modification of the compound of formula (1) with a content of water < 17% and ≥ 13% obtained by Example 3.

Table 3

| d-spacing (Å) | Relative intensity | d-spacing (Å) | Relative intensity | d-spacing (Å) | Relative intensity |
|---|---|---|---|---|---|
| 21.8 | w | 7.2 | m | 4.75 | w |
| 11.6 | s | 7.0 | w | 4.49 | w |
| 11.0 | w | 5.77 | s | 4.01 | w |
| 9.2 | s | 5.51 | s | 3.86 | m |

(continued)

| d-spacing (Å) | Relative intensity | d-spacing (Å) | Relative intensity | d-spacing (Å) | Relative intensity |
|---|---|---|---|---|---|
| 7.3 | m | 4.93 | s | 3.65 | vs |

Fig. 3. shows the powder X-ray diffraction pattern of the compound of formula (1)) with a content of water < 17% and ≥ 13%, obtained from Example 3.

The invention relates also to a new crystal modification of the compound of formula (1) with a content of water < 13% and > 0%, characterized by peaks at about about 21.8, 11.0, 9.0, and 7.3 d-spacing units in its powder X-ray diffraction pattern.

Specifically, the new crystal modification of the compound of formula (1) with a content of water < 13% and > 0%, characterized by peaks at about about 21.8, 11.5, 11.0, 9.0, 7.3 and 3.58 d-spacing units in its powder X-ray diffraction pattern.

Table 4 shows the characteristic spacing between the lattice planes designated by d and expresses in Angstöm units [A] and their corresponding characteristic relative intensisty (very weak, weak, medium, strong or very strong) of the new crystal modification of the compound of formula (1) with a content of water < 13% and > 0% obtained by Example 4.

Table 4

| d-spacing (Å) | Relative intensity |
|---|---|
| 21.8 | vw |
| 11.5 | m |
| 11.0 | w |
| 9.0 | m |
| 7.3 | w |
| 3.58 | vs |

Fig. 4. shows the powder X-ray diffraction pattern of the new crystal modification of the compound of formula (1) with a content of water < 13% and > 0%, obtained from Example 4.

A further embodiment of the present invention relates to a crystal modification of the compound of formula (1')

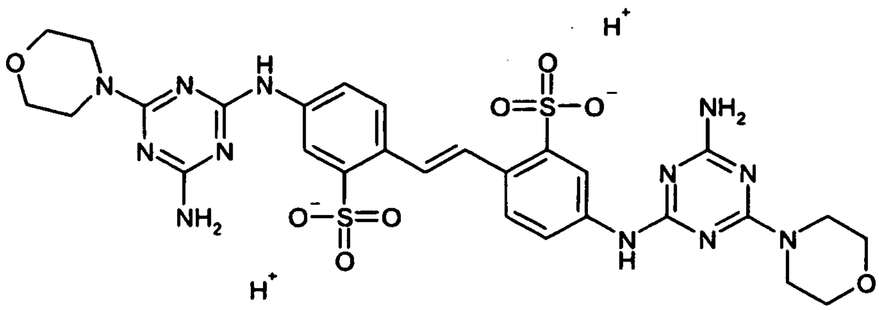

(1'),

characterized by
peaks at about 10.1, 5.62, 3.55 and 3.37 d-spacing units in its powder X-ray diffraction pattern at a content of water > 0%.

This compound of formula (1') is obtainable by the reaction of a compound of formula (1) with a protonic acid, i.e. HCl.

Specifically, the new crystal modification of the compound of formula (1') is characterized by peaks at about 10.1, 5.92, 5.62, 4.21, 3.72, 3.55 and 3.37 d-spacing units in its powder X-ray diffraction pattern.

More specifically, the new crystal modification of the compound of formula (1') is characterized by peaks at about 10.1, 5.92, 5.62, 5.05, 4.77, 4.38, 4.21, 9.97, 3.89, 3.72, 3.55, 3.37, 3.06, 2.76, 2.59 and 2.4 d-spacing units in its powder X-ray diffraction pattern.

Table 5 shows the characteristic spacing between the lattice planes designated by d and expresses in Angstöm units [A] and their corresponding characteristic relative intensisty (very weak, weak, medium, strong or very strong) of

the new crystal modification of the compound of formula (1') obtained by Example 5.

Table 5

| d-spacing (Å) | Relative intensity | d-spacing (Å) | Relative intensity |
|---|---|---|---|
| 10.1 | s | 3.37 | s |
| 9.5 | vw | 3.24 | vw |
| 5.96 | m | 3.19 | vw |
| 5.62 | s | 3.06 | w |
| 5.05 | w | 2.84 | vw |
| 4.77 | w | 2.76 | w |
| 4.38 | w | 2.70 | vw |
| 4.21 | m | 2.59 | w |
| 3.97 | w | 2.52 | vw |
| 3.89 | w | 2.45 | vw |
| 3.72 | m | 2.4 | w |
| 3.55 | vs | | |

Fig. 5. shows the powder X-ray diffraction pattern of the compound of formula (1') obtained from Example 5.
The invention relates also to a crystal modification of the mixture comprising (1)

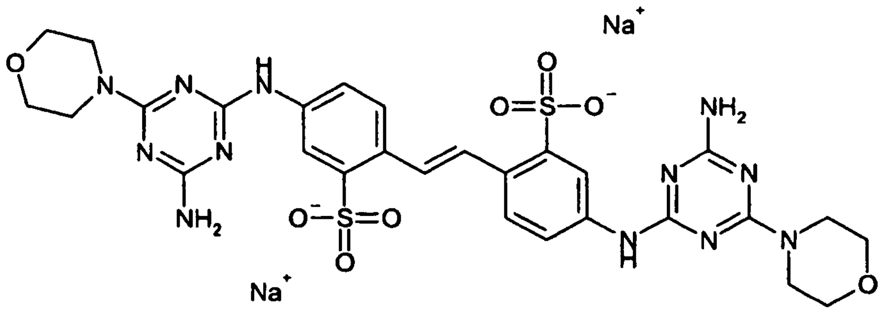
(1)

and
a compound (1a)

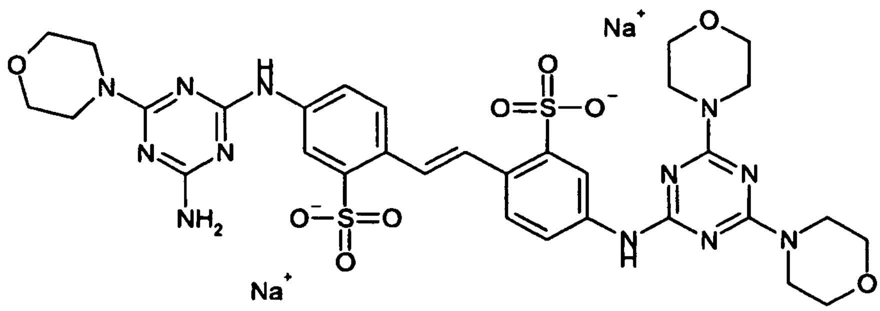
(1a)

characterized by
peaks at about 21.8, 11.0 and 7.3 d-spacing units in its powder X-ray diffraction pattern at a content of water > 0%.
Specifically, the new crystal modification of the mixture with a content of water ≥ 19% is characterized by peaks

at about 21.8, 11.8, 11.0, 9.2, 7.3, 5.82, 4.48, 4.37, 4.05, 3.96, 3.88, 3.36, 2.95 and 2.56 d-spacing units in its powder X-ray diffraction pattern.

Specifically, the new crystal modification of the mixture with a content of water < 19% and ≥ 17% is characterized by peaks at about 21.8, 11.7, 11.5, 11.0, 9.2, 7.3, 7.2, 7.0, 5.78, 5.56, 4.95, 4.75, 4.49, 4.04, 3.87 and 3.71 d-spacing units in its powder X-ray diffraction pattern.

Specifically, the new crystal modification of the mixture with a content of water < 17% and 2: 13% is characterized by peaks at about 21.8, 11.6, 11.0, 9.2, 7.3, 7.2, 5.77, 5.51, 4.93, 3.86 and 3.65 d-spacing units in its powder X-ray diffraction pattern.

Specifically, the new crystal modification of the mixture with a content of water < 13% and ≥ 0% is characterized by peaks at about 21.8, 11.5, 11.0, 9.0, 7.3 and 3.58 d-spacing units in its powder X-ray diffraction pattern.

The molar ratio of the mixture of compound (1) : compound (1a) is in the range of 99.99:0.01 to 10:90. Preferably, the molar ratio of the mixture of compound (1) : compound (1a) is in the range of 99.99:0.01 to 30:70. More preferably, the molar ratio of the mixture of compound (1) : compound (1a) is in the range of 99.99:0.01 to 50:50. Especially, the molar ratio of the mixture of compound (1) : compound (1a) is in the range of 99.99:0.01 to 90:10.

The invention relates also to a crystal modification of the mixture comprising (1')

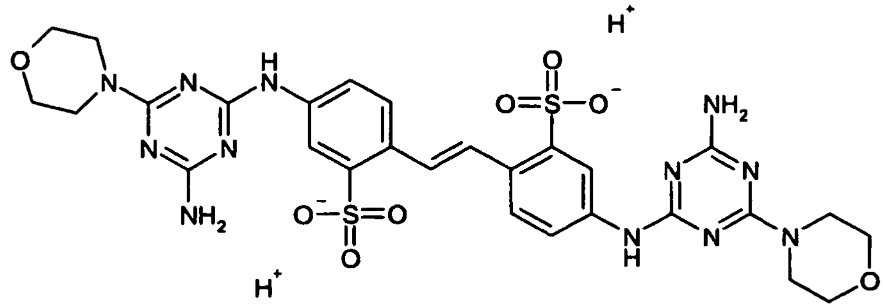

(1')

and

a compound (1'a)

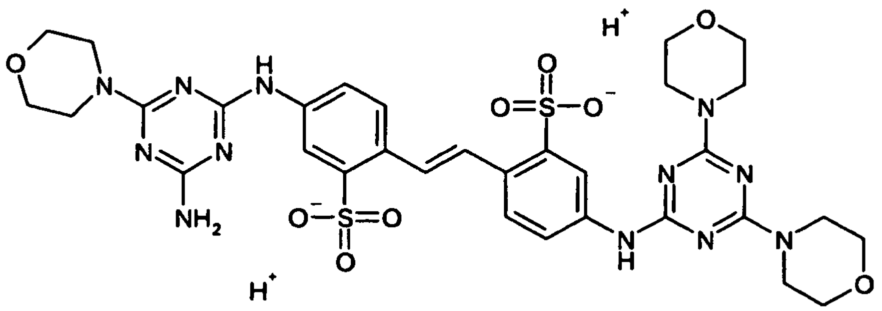

(1'a)

characterized by

peaks at about 10.1, 5.62, 3.55 and 3.37 d-spacing units in its powder X-ray diffraction pattern at a content of water > 0%.

More specifically, the new crystal modification of the mixture of compund (1') and (1'a) is characterized by peaks at about 10.1, 5.92, 5.62, 5.05, 4.77, 4.38, 4.21, 9.97, 3.89, 3.72, 3.55, 3.37, 3.06, 2.76, 2.59 and 2.4 d-spacing units in its powder X-ray diffraction pattern.

The molar ratio of the mixture of compound (1') : compound (1'a) is in the range of 99.99:0.01 to 10:90.

Preferably, the molar ratio of the mixture of compound (1'): compound (1'a) is in the range of 99.99:0.01 to 30:70. More preferably, the molar ratio of the mixture of compound (1') : compound (1'a) is in the range of 99.99:0.01 to 50:50. Especially, the molar ratio of the mixture of compound (1') : compound (1'a) is in the range of 99.99:0.01 to 90:10.

A further embodiment of the present invention also relates to a mixture comprising

(a) the crystal modification of the compound of formula (1) or the crystal modification of the mixture of the compound of formula (1) and the compound of formula (1a) and

(b) at least one compound of formula (2)

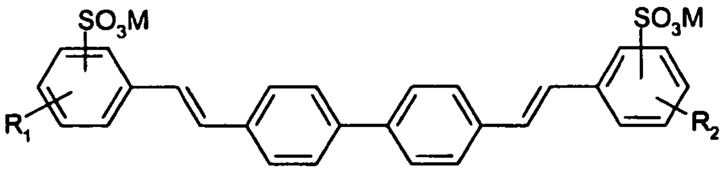

(2),

wherein
$R_1$ and $R_2$, independently of each other, are hydrogen, $C_1$-$C_8$alkyl, $C_1$-$C_8$alkoxy or halogen, and M is hydrogen or a cation.

A further embodiment of the present invention also relates to a mixture comprising

(a) the crystal modification of formula (1') or the crystal modification of the mixture of the compound of formula (1') and the compound of formula (1'a) and
(b) at least one compound of formula (2)

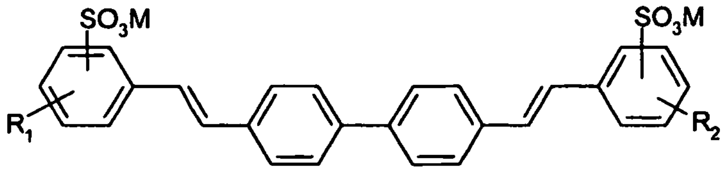

(2),

wherein
$R_1$ and $R_2$, independently of each other, are hydrogen, $C_1$-$C_8$alkyl, $C_1$-$C_8$alkoxy or halogen, and M is hydrogen or a cation.

The cation M is preferably an alkali metal atom, an alkaline earth metal atom, ammonium or a cation formed from an amine. Preferred are Na, K, Ca, Mg, ammonium, mono-, di-, tri- or tetra-$C_1$-$C_4$alkylammonium, mono-, di- or tri-$C_2$-$C_4$-hydroxyalkylammonium or ammonium that is di- or tri-substituted with a mixture of $C_1$-$C_4$-alkyl and $C_2$-$C_4$-hydroxyalkyl groups. Highly preferred is sodium.

Within the scope of the above definitions, $C_1$-$C_8$alkyl may be methyl, ethyl, n- or isopropyl, n-, sec.- or t-butyl, or linear or branched pentyl, hexyl, heptyl or octyl. Preferred are $C_1$-$C_4$alkyl groups.

Within the scope of the above definitions, $C_1$-$C_8$alkoxy may be methoxy, ethoxy, propoxy, isopropoxy, butoxy, isobutoxy, sec.-butoxy, tert.-butoxy, or linear or branched higher alkoxy groups. Preferred are $C_1$-$C_4$alkoxy groups, especially methoxy or ethoxy. Highly preferred is methoxy.

Halogen may be fluorine, chlorine, bromine or iodine, preferably chlorine.

The compounds of formula (2) are known and can be prepared in analogy to known processes.

In the mixtures of compounds of formulae (1) or (1') and (2) the molar ratio of the new crystal modification of the compound of formula (1) or (1') to compound (2) is usually in the range of from 0.1:99.9 to 99.9:0.1, preferably from 1:99 to 99:1 and more preferably from 5:95 to 95:5. Highly preferred is a molar ratio of from 10:90 to 90: 1 0, especially 20:80 to 80:20. Most important is a molar ratio of from 30:70 to 70:30, especially 40:60 to 60:40.

The crystal modification of the compound of formula (1) and/or the crystal modification of formula (1') or the crystal modification of the mixture of the compound of formula (1) and the compound of formula (1a) or the crystal modification of the mixture of the compound of formula (1') and the compound of formula (1'a)) and at least one compound according to formula (2) as defined above are useful as fluorescent whitening agent for paper or textile material. It can be used for example in detergent compositions.

Therefore, a further embodiment of the present invention relates to detergent composition comprising the new crystal modification of the compound of formula (1).

Therefore, a further embodiment of the present invention relates to detergent composition comprising the new crystal modification of the compound of formula (1').

A further embodiment of the present invention relates to detergent composition comprising a mixture of the crystal modification of the compound of formula (1) or the crystal modification of the mixture of the compound of formula (1) and the compound of formula (1 a) and at least one compound according to formula (2) as defined above.

A further embodiment of the present invention relates to detergent composition comprising a mixture of crystal modification of formula (1') or the crystal modification of the mixture of the compound of formula (1') and the compound of formula (1'a) and at least one compound according to formula (2) as defined above.

A further embodiment of the present invention is a detergent composition preferably comprising

i) 1 - 70% of an anionic surfactant and/or a nonionic surfactant;
ii) 0 - 75% of a builder;
iii) 0 - 30% of a peroxide;
iv) 0 - 10% of a peroxide activator; and
v) 0.001 - 5% of the crystal modification of the compound of formula (1) or the crystal modification of the mixture of the compound of formula (1) and at least one compound according to formula (2) as defined above,
each by weight, based on the total weight of the detergent composition.

A further embodiment of the present invention is a detergent composition preferably comprising

i) 1 - 70% of an anionic surfactant and/or a nonionic surfactant;
ii) 0 - 75% of a builder;
iii) 0 - 30% of a peroxide;
iv) 0 -10% of a peroxide activator; and
v) 0.001 - 5% of the crystal modification of formula (1') or the crystal modification of the mixture of the compound of formula (1') and the compound of formula (1'a) and at least one compound according to formula (2) as defined above,
each by weight, based on the total weight of the detergent composition.

More preferably the detergent composition comprises

i) 5 - 70% of an anionic surfactant and/or a nonionic surfactant;
ii) 5 - 70% of a builder;
iii) 0.5 - 30% of a peroxide;
iv) 0.5 - 10% of a peroxide activator and/or 0.1 - 2% of a bleaching catalyst; and
v) 0.01 - 5% of the crystal modification of the compound of formula (1) or the crystal modification of the mixture of the compound of formula (1) and the compound of formula (1a) and at least one compound according to formula (2) as defined above,
each by weight, based on the total weight of the detergent composition.

More preferably the detergent composition comprises

i) 5 - 70% of an anionic surfactant and/or a nonionic surfactant;
ii) 5 - 70% of a builder;
iii) 0.5 - 30% of a peroxide;
iv) 0.5 - 10% of a peroxide activator and/or 0.1 - 2% of a bleaching catalyst; and
v) 0.01 - 5% of the crystal modification of formula (1') or the crystal modification of the mixture of the compound of formula (1') and the compound of formula (1'a) and at least one compound according to formula (2) as defined above,
each by weight, based on the total weight of the detergent composition.

Especially preferably the detergent composition comprises

i) 5 - 70% of an anionic surfactant and/or a nonionic surfactant;
ii) 5 - 70% of a builder;
iii) 0.5 - 30% of a peroxide;
iv) 0.5 - 10% of a peroxide activator and/or 0.1 - 2% of a bleaching catalyst; and
v) 0.01 - 5% of the crystal modification of the compound of formula (1) or the crystal modification of the mixture of the compound of formula (1) and the compound of formula (1a) and the compound according to formula (2a)

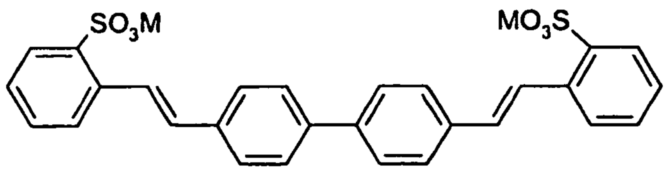

(2),

wherein M is hydrogen, an alkaline- or alkaline earth-metal, or ammonium,

each by weight, based on the total weight of the detergent composition.

Especially preferably the detergent composition comprises

i) 5 - 70% of an anionic surfactant and/or a nonionic surfactant;
ii) 5 - 70% of a builder;
iii) 0.5 - 30% of a peroxide;
iv) 0.5 -10% of a peroxide activator and/or 0.1 - 2% of a bleaching catalyst; and
v) 0.01 - 5% of the crystal modification of formula (1') or the crystal modification of the mixture of the compound of formula (1') and the compound of formula (1'a) and the compound according to formula (2a)

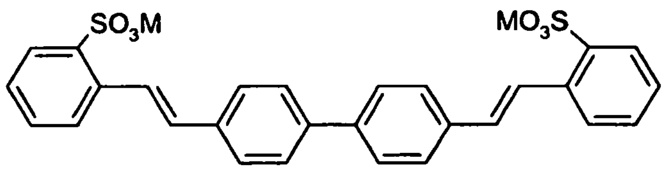

**(2)**,

wherein M is hydrogen, an alkaline- or alkaline earth-metal, or ammonium,
each by weight, based on the total weight of the detergent composition.

In general, the crystal modification of the compound of formula (1) and/or the crystal modification of formula (1') and/or the crystal modification of the mixture of the compound of formula (1) and the compound of formula (1a) and/or the crystal modification of the mixture of the compound of formula (1') and the compound of formula (1'a) and at least one compound according to formula (2) as defined above, is used in an amount of 0.001 - 5%, especially in an amount of 0.01 - 5%. Highly preferred is an amount of 0.05 - 5%, especially 0.05 - 2%.
In general, amounts given in percent are to be understood as being percent by weight, based on the total weight, unless otherwise stated.

The detergent may be formulated as a solid, as an aqueous liquid comprising, e.g., 5 - 50, preferably 10 - 35% water or as a non-aqueous liquid detergent, containing not more than 5, preferably 0 - 1 wt% of water, and based on a suspension of a builder in a non-ionic surfactant, as described, e.g., in GB-A-2158454.

The anionic surfactant component may be, e.g., an alkylbenzenesulfonate, an alkylsulfate, an alkylethersulfate, an olefinsulfonate, an alkanesulfonate, a fatty acid salt, an alkyl or alkenyl ether carboxylate or an $\alpha$-sulfofatty acid salt or an ester thereof. Preferred are alkylbenzenesulfonates having 10 to 20 carbon atoms in the alkyl group, alkylsulfates having 8 to 18 carbon atoms, alkylethersulfates having 8 to 18 carbon atoms, and fatty acid salts being derived from palm oil or tallow and having 8 to 18 carbon atoms. The average molar number of ethylene oxide added in the alkylethersulfate is preferably 1 to 20, preferably 1 to 10. The salts are preferably derived from an alkaline metal like sodium and potassium, especially sodium. Highly preferred carboxylates are alkali metal sarcosinates of formula $R\text{-}CO(R^1)CH_2COOM^1$ in which R is alkyl or alkenyl having 9-17 carbon atoms in the alkyl or alkenyl radical, $R^1$ is $C_1$-$C_4$ alkyl and $M^1$ is alkali metal, especially sodium.
The nonionic surfactant component may be, e.g., primary and secondary alcohol ethoxylates, especially the $C_8$-$C_{20}$ aliphatic alcohols ethoxylated with an average of from 1 to 20 moles of ethylene oxide per mole of alcohol, and more especially the $C_{10}$-$C_{15}$ primary and secondary aliphatic alcohols ethoxylated with an average of from 1 to 10 moles of ethylene oxide per mole of alcohol. Non-ethoxylated nonionic surfactants include alkylpolyglycosides, glycerol monoethers, and polyhydroxyamides (glucamide).

The total amount of anionic surfactant and nonionic surfactant is preferably 5 - 50% by weight, preferably 5 - 40% by weight and more preferably 5 - 30% by weight. As to these surfactants it is preferred that the lower limit is 10% by weight.

The builder component may be an alkali metal phosphate, especially a tripolyphosphate; a carbonate or bicarbonate, especially the sodium salts thereof; a silicate or disilicate; an aluminosilicate; a polycarboxylate; a polycarboxylic acid; an organic phosphonate; or an aminoalkylene poly (alkylene phosphonate); or a mixture of these.
Preferred silicates are crystalline layered sodium silicates of the formula $NaHSi_mO_{2m+1}.pH_2O$ or $Na_2Si_mO_{2m+1}.pH_2O$ in which m is a number from 1.9 to 4 and p is 0 to 20.

Preferred aluminosilicates are the commercially-available synthetic materials designated as Zeolites A, B, X, and HS, or mixtures of these. Zeolite A is preferred.
Preferred polycarboxylates include hydroxypolycarboxylates, in particular citrates, polyacrylates and their copolymers with maleic anhydride.
Preferred polycarboxylic acids include nitrilotriacetic acid and ethylene diamine tetra-acetic acid.

Preferred organic phosphonates or aminoalkylene poly (alkylene phosphonates) are alkali metal ethane 1-hydroxy diphosphonates, nitrilo trimethylene phosphonates, ethylene diamine tetra methylene phosphonates and diethylene triamine penta methylene phosphonates.
The amount of builders is preferably 5 - 70% by weight, preferably 5 - 60% by weight and more preferably 10 - 60% by weight. As to the builders it is preferred that the lower limit is 15% by weight, especially 20% by weight.

Suitable peroxide components include, for example, the organic and inorganic peroxides known in the literature and available commercially that bleach textile materials at conventional washing temperatures, for example at from 5 to 95°C.
In particular, the organic peroxides are, for example, monoperoxides or polyperoxides having alkyl chains of at least 1, preferably 1 to 20, carbon atoms; in particular diperoxydicarboxylates having 1 to 12 C atoms, such as diperoxyperazelates, peroxyacetic acid, diperoxypersebacates, diperoxyphthalates, ε-phthalimido peroxyhexanoic acid and/or diperoxydodecanedioates, especially their corresponding free acids, are of interest. It is preferred, however, to employ very active inorganic peroxides, such as persulphate, perborate and/or percarbonate. It is, of course, also possible to employ mixtures of organic and/or inorganic peroxides.

The amount of peroxide is preferably 0.5 - 30% by weight, preferably 1 - 20% by weight and more preferably 1 -15% by weight. In case a peroxide is used, the lower limit is preferably 2% by weight, especially 5% by weight.

The peroxides, especially the inorganic peroxides, are preferably activated by the inclusion of a bleach activator. Preferred are such compounds that, under perhydrolysis conditions, yield unsubstituted or substituted perbenzo- and/or peroxo-carboxylic acids having from 1 to 10 carbon atoms, especially from 2 to 4 carbon atoms. Suitable compounds include those that carry O- and/or N-acyl groups having the said number of carbon atoms and/or unsubstituted or substituted benzoyl groups. Preference is given to polyacylated alkylenediamines, especially tetraacetylethylenediamine (TAED), acylated glycolurils, especially tetraacetylglycoluril (TAGU), N,N-diacetyl-N,N-dimethyl-urea (DDU), acylated triazine derivatives, especially 1,5-diacetyl-2,4-dioxohexahydro-1,3,5-triazine (DADHT), compounds of formula

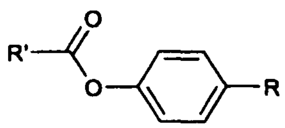

wherein R is a sulfonate group, a carboxylic acid group or a carboxylate group, and wherein R' is linear or branched ($C_7$-$C_{15}$)alkyl; also activators that are known under the names SNOBS, SLOBS, NOBS and DOBA, acylated polyhydric alcohols, especially triacetin, ethylene glycol diacetate and 2,5-diacetoxy-2,5-dihydrofuran and acetylated sorbitol and mannitol and acylated sugar derivatives, especially pentaacetylglucose (PAG), sucrose polyacetate (SUPA), pentaacetylfructose, tetraacetylxylose and octaacetyllactose, and acetylated, optionally N-alkylated, glucamine and gluconolactone. The combinations of conventional bleach activators disclosed in German Patent Application DE-A-44 43 177 may also be used. Nitrile compounds that form peroxyimidic acids with peroxides are also suitable as bleach activators. Preferred are tetraacetyl ethylenediamine and nonoyloxybenzene sulfonate.

The amount of bleach activator is preferably 0 - 10% by weight, preferably 0-8% by weight. In case a bleach activator is used, the lower limit is preferably 0.5% by weight, especially 1% by weight.

Bleaching catalysts which may be added include, e.g., enzymatic peroxide precursors and/or metal complexes. Preferred metal complexes are manganese, cobalt or iron complexes such as manganese or iron phthalocyanines or the complexes described in EP-A-0509787. In case a bleaching catalyst is used the amount is preferably 0.1 - 2% by weight.

Furthermore, the detergent can optionally contain enzymes. Enzymes can be added to detergents for stain removal. The enzymes usually improve the performance on stains that are either protein- or starch-based, such as those caused by blood, milk, grass or fruit juices. Preferred enzymes are cellulases, proteases, amylases and lipases. Preferred enzymes are cellulases and proteases, especially proteases. Cellulases are enzymes which act on cellulose and its derivatives and hydrolyze them into glucose, cellobiose, cellooligosaccharide. Cellulases remove dirt and have the effect of mitigating the roughness to the touch. Examples of enzymes to be used include, but are by no means limited to, the following:

proteases as given in US-B-6,242,405, column 14, lines 21 to 32;
lipases as given in US-B-6,242,405, column 14, lines 33 to 46;
amylases as given in US-B-6,242,405, column 14, lines 47 to 56; and
cellulases as given in US-B-6,242,405, column 14, lines 57 to 64.

The enzymes can optionally be present in the detergent. When used, the enzymes are usually present in an amount of 0.01 - 5% by weight, preferably 0.05 - 5% and more preferably 0.1-4% by weight, based on the total weight

of the detergent.

Further preferred additives for the detergents according to the invention are polymers that, during the washing of textiles, inhibit staining caused by dyes in the washing liquor that have been released from the textiles under the washing conditions (dye fixing agents, dye transfer inhibitors). Such polymers are preferably polyvinylpyrrolidones, polyvinylimidazoles or polyvinylpyridine N-oxides which may have been modified by the incorporation of anionic or cationic substituents, especially those having a molecular weight in the range from 5000 to 60 000, more especially from 10 000 to 50 000. Such polymers are usually used in an amount of from 0.01 to 5 %, preferably 0.05 to 5 % by weight, especially 0.1 to 2 % by weight, based on the total weight of the detergent. Preferred polymers are those given in WO-A-02/02865 (see especially page 1, last paragraph and page 2, first paragraph).

The detergents used will usually contain one or more auxiliaries such as soil suspending agents, for example sodium carboxymethylcellulose; salts for adjusting the pH, for example alkali or alkaline earth metal silicates; foam regulators, for example soap; salts for adjusting the spray drying and granulating properties, for example sodium sulphate; perfumes; and also, if appropriate, antistatic and softening agents; such as smectite clays; photobleaching agents; pigments; and/or shading agents. These constituents should, of course, be stable to any bleaching system employed. Such auxiliaries can be present in an amount of, for example, 0.1 to 20% by weight, preferably 0.5 to 10 % by weight, especially 0.5 to 5 % by weight, based on the total weight of the detergent.

The detergent compositions can take a variety of physical forms including powder, granular, tablet and liquid forms. Examples thereof are conventional powder heavy-duty detergents, compact and supercompact heavy-duty detergents and tablets, like heavy-duty detergent tablets. One important physical form is the so-called concentrated granular form adapted to be added to a washing machine.

Of importance are also the so-called compact (or supercompact) detergents. In the field of detergent manufacture, a trend has developed recently towards the production of compact detergents which contain increased amounts of active substance. In order to minimize energy expenditure during the washing process, the compact detergents are required to operate efficiently at temperatures as low as 40°C, or even at room temperatures, e.g. at 25°C. Such detergents usually contain only low amounts of fillers or processing aids, like sodium sulfate or sodium chloride. The amount of such fillers is usually 0-10% by weight, preferably 0 - 5% by weight, especially 0 - 1 % by weight, based on the total weight of the detergent. Such detergents usually have a bulk density of 650 - 1000 g/), preferably 700 - 1000 g/l and especially 750 -1000 g/l.

The detergents can also be present in the form of tablets. Relevant characteristics of tablets are ease of dispensing and convenience in handling. Tablets are the most compact delivery of solid detergents and have a bulk density of, for example, 0.9 to 1.3 kg/litre. To enable fast disintegration laundry detergent tablets generally contain special disintegrants:

- Effervescents such as carbonate/hydrogencarbonate/citric acid;
- swelling agents like cellulose, carboxymethyl cellulose, cross-linked poly(N-vinylpyrrollidone);
- quickly dissolving materials such as Na (K) acetate, or Na (K) citrate;
- rapidly dissolving water-soluble rigid coating such as dicarboxy acids.

The tablets can also contain combinations of any of the above disintegrants.

The detergent may also be formulated as an aqueous liquid comprising 5 - 50, preferably 10 - 35% water or as a non-aqueous liquid detergent, containing not more than 5, preferably 0 - 1wt.% of water. Non-aqueous liquid detergent compositions can contain other solvents as carriers. Low molecular weight primary or secondary alcohols exemplified by methanol, ethanol, propanol, and isopropanol are suitable. Monohydric alcohols are preferred for solubilizing surfactant, but polyols such as those containing from 2 to about 6 carbon atoms and from 2 to about 6 hydroxy groups (e.g., 1,3-propanediol, ethylene glycol, glycerine, and 1,2-propanediol) can also be used. The compositions may contain from 5%
to 90%, typically 10% to 50% of such carriers. The detergents can also be present as the so-called "unit liquid dose" form.

This detergent treatment of textiles can be conducted as a domestic treatment in normal washing machines.

The textile fibres treated may be natural or synthetic fibres or mixtures thereof. Examples of natural fibres include vegetable fibres such as cotton, viscose, flax, rayon or linen, preferably cotton and animal fibres such as wool, mohair, cashmere, angora and silk, preferably wool. Synthetic fibres include polyester, polyamide and polyacrylonitrile fibres. Preferred textile fibres are cotton, polyamide and wool fibres, especially cotton fibres. Preferably, textile fibres treated according to the method of the present invention have a density of less than 200 $g/m^2$.

According to this process usually an amount of 0.01 to 3.0% by weight, especially 0.05 to 3.0% by weight, based on the weight of the textile fibre material, of the crystal modification of the compound of formula (1) and/or the crystal modification of formula (1') and/or the crystal modification of the mixture of the compound of formula (1) and the compound of formula (1a) and/or the crystal modification of the mixture of the compound of formula (1') and the compound of formula (1'a) and at least one compound according to formula (2) as defined above, is used.

The process is usually conducted in the temperature range of from 5 to 100°C, especially 5 to 60°C. Preferred is a temperature range of 5 to 40°C, especially 5 to 35°C and more preferably 5 to 30°C.

The detergent compositions herein will preferably be formulated such that, during use in aqueous cleaning operations, the wash water will have a pH of between about 6.5 and about 11, preferably between about 7.5 and 11. Laundry products are typically at pH 9 - 11. Techniques for controlling pH at recommended usage levels include the use of buffers, alkalis, acids, etc., and are well known to those skilled in the art.

Machine laundry methods herein typically comprise treating soiled laundry with an aqueous wash solution in a washing machine having dissolved or dispensed therein an effective amount of a machine laundry detergent composition in accordance with the invention. By an effective amount of the detergent composition it is meant, e.g., from 20 g to 300 g of product dissolved or dispersed in a wash solution of volume from 5 to 85 litres, as are typical product dosages and wash solution volumes commonly employed in conventional machine laundry methods. Examples are

- top-loading, vertical axis U.S.-type automatic washing machines using about 45 to 83 liters of water in the wash bath, a wash cycle of about 10 to about 14 minutes and a wash water temperature of about 10 to about 50°C;
- front-loading, horizontal-axis European-type automatic washing machine using about 8 to 15 liters of water in the wash bath, a wash cycle of about 10 to about 60 minutes and a wash water temperature of about 30 to about 95°C;
- top-loading, vertical-axis Japanese-type automatic washing machine using about 26 to 52 liters of water in the wash bath, a wash cycle of about 8 to about 15 minutes and a wash water temperature of about 5 to about 25°C.

The liquor ratio is preferably 1:4 to 1:40, especially 1:4 to 1:15. Highly preferred is a liquor ratio of 1:4 to 1:10, especially 1:5 to 1:9.

Preferred are detergent compositions comprising

i) 1 - 70% of an anionic surfactant and/or a nonionic surfactant;
ii) 0 - 75% of a builder;
iii) 0 - 30% of a peroxide;
iv) 0 -10% of a peroxide activator;
v) 0.001 - 5% of the crystal modification of the compound of formula (1) or the crystal modification of the mixture of the compound of formula (1) and the compound of formula (1a) and at least one compound according to formula (2) as defined above; and
vi) 0.05 - 5% of at least one enzyme selected from the group consisting of cellulase, protease, amylase and lipase, especially protease.

Preferred are detergent compositions comprising

i) 1 - 70% of an anionic surfactant and/or a nonionic surfactant;
ii) 0 - 75% of a builder;
iii) 0 - 30% of a peroxide;
iv) 0 - 10% of a peroxide activator;
v) 0.001 - 5% of the crystal modification of formula (1') or the crystal modification of the mixture of the compound of formula (1') and the compound of formula (1'a) and at least one compound according to formula (2) as defined above; and
vi) 0.05 - 5% of at least one enzyme selected from the group consisting of cellulase, protease, amylase and lipase, especially protease.

Highly preferred are detergent compositions comprising

i) 5 - 70% of an anionic surfactant and/or a nonionic surfactant;
ii) 5 - 70% of a builder;
iii) 0.5 - 30% of a peroxide;
iv) 0.5 - 10% of a peroxide activator and/or 0.1-2% of a bleaching catalyst;
v) 0.01 - 5% of the new crystal modification of the compound of formula (1) or a mixture of the new crystal modification of the compound of formula (1) and at least one compound according to formula (2) as defined above; and
vi) 0.05 - 5% of at least one enzyme selected from the group consisting of cellulase, protease, amylase and lipase, especially protease.

Highly preferred are detergent compositions comprising

i) 5 - 70% of an anionic surfactant and/or a nonionic surfactant;
ii) 5 - 70% of a builder;
iii) 0.5 - 30% of a peroxide;
iv) 0.5 - 10% of a peroxide activator and/or 0.1-2% of a bleaching catalyst;
v) 0.01 - 5% of of the crystal modification of formula (1') or the crystal modification of the mixture of the compound of formula (1') and the compound of formula (1'a) and at least one compound according to formula (2) as defined above; and
vi) 0.05 - 5% of at least one enzyme selected from the group consisting of cellulase, protease, amylase and lipase, especially protease.

Especially preferred are detergent compositions comprising

i) 5 - 70% of an anionic surfactant and/or a nonionic surfactant;
ii) 5 - 70% of a builder;
iii) 0.5 - 30% of a peroxide;
iv) 0.5 -10% of a peroxide activator and/or 0.1-2% of a bleaching catalyst;
v) 0.01 - 5% % of the crystal modification of the compound of formula (1) or the crystal modification of the mixture of the compound of formula (1) and the compound of formula (1a) and the compound according to formula (2a)

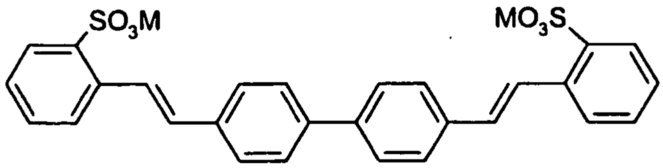

(2a),

wherein M is hydrogen, an alkaline- or alkaline earth-metal, or ammonium; and
vi) 0.05 - 5% of at least one enzyme selected from the group consisting of cellulase, protease, amylase and lipase, especially protease.

Especially preferred are detergent compositions comprising

i) 5 - 70% of an anionic surfactant and/or a nonionic surfactant;
ii) 5 - 70% of a builder;
iii) 0.5 - 30% of a peroxide;
iv) 0.5 -10% of a peroxide activator and/or 0.1-2% of a bleaching catalyst;
v) 0.01 - 5% of the crystal modification of formula (1') or the crystal modification of the mixture of the compound of formula (1') and the compound of formula (1'a) and the compound according to formula (2a)

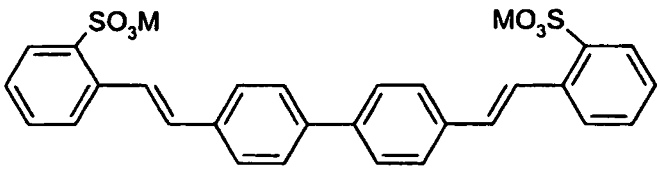

(2a),

wherein M is hydrogen, an alkaline- or alkaline earth-metal, or ammonium; and
vi) 0.05 - 5% of at least one enzyme selected from the group consisting of cellulase, protease, amylase and lipase, especially protease.

A further object of the present invention is to provide a process for the domestic washing treatment of a textile fibre material wherein the textile fibre material is contacted with an aqueous solution of a detergent composition of the crystal modification of the compound of formula (1) and/or the crystal modification of formula (1') and/or the crystal modification of the mixture of the compound of formula (1) and the compound of formula (1a) and/or the crystal modification of the mixture of the compound of formula (1') and the compound of formula (1'a) and at least one compound according to formula (2) as defined above,
wherein the temperature of the solution is between 5°C and 40°C, preferably between 5°C and 30°C, throughout the

process.

The compounds and mixtures used according to the present invention are particularly advantageous in that they exhibit not only extremely high whitening ability, but, in addition, in many cases highly desirable water solubilities and also possess excellent white aspects in the solid state. A further advantage of the present invention is that the detergent composition delivers improved whiteness performance and fabric feel. Furthermore the compounds and especially the mixtures show very good results with respect to exhaustion properties.

The compounds have the advantage that they are also effective in the presence of active chlorine donors, such as, for example, hypochlorite and can be used without substantial loss of the effects in washing baths with non-ionic washing agents, for example alkylphenol polyglycol ethers. Also in the presence of perborate or peracids and activators, for example tetraacetylglycoluril or ethylenediamine-tetraacetic acid are the compounds and mixtures of compounds stable both in pulverulent washing agent and in washing baths. In addition, they impart a brilliant appearance in daylight.

A further embodiment of the present invention is a method of producing the new crystal modification of the compound of formula (1) and of the mixture of the compound of formula (1) and the compound of formula (1a), characterized by reacting in a first step 4,4'-diaminostilbene-2,2'-disulfonic in its sodium salt form, sodium carbonate, methylethylketone and cyanurchloride at low temperature at a pH of between 4 and 5; in a second step morpholino is added and the pH is increased to 8.5 to 9.5 and the temperature is increase to higher temperature (60°C-110°C) and in a third step the reaction solution is cooled down to lower temperature (10-15°C) and the obtained precipitation of the new crystal modification of formula (1) is filtered off and washed by conventional processes.

A further embodiment of the present invention is a method of producing the new crystal modification of the compound of formula (1') and of the mixture of the compound of formula (1') and the compound of formula (1'a), characterized by reacting in a first step 4,4'-diaminostilbene-2,2'-disulfonic in its sodium salt form, sodium carbonate, methylethylketone and cyanurchloride at low temperature at a pH of between 4 and 5; in a second step morpholino is added and the pH is increased to 8.5 to 9.5 and the temperature is increase to higher temperature (60°C - 110°C), whereby an acid, such as HCl is added until a pH of 4 is reached and in a third step the reaction solution is cooled down to lower temperature (10 - 15°C) and the obtained precipitation of the new crystal modification of formula (1') is filtered off and washed by conventional processes.

The following Examples serve to illustrate the invention; parts and percentages are by weight, unless otherwise stated.

**Example 1**

A solution consisting of 400 ml water, 45 g of 4,4'-diaminostilbene-2,2'-disulfonic in its sodium salt form and 11.5 g sodium carbonate is added to a mixture consisting of 260 g of methylethylketone, 225 g of ice and 45 g of cyanurchloride. During the addition, the reaction temperature is kept below 10°C by external cooling and the pH is kept between 4.5 and 5 by the addition of 3 g sodium carbonate in 20 ml water. Afterwards 28.6 ml of a 25% ammonia solution are added and the pH value is adjusted between 9.0 and 9.2 by the addition of 6.8 g sodium hydroxide in 35 g water. Afterwards, the reaction solution is stirred for 1 h at 45°C. Then 23 g morpholine is added and the reaction solution is heated up to 70°C. Simultaneously, the pH is adjusted to a value between 8.8 and 9.2 by the addition of 12.5 g sodiumhydroxyd in 70 g water. The reaction solution is stirred for 2 h at 70°C.

Afterwards the reaction solution is heated up to 98°C whereby 320 ml of a mixture of methylethylketone and water are distilled off. The remaining reaction solution is cooled to 20°C, stirred for 4 h and the precipitation is filtered off.

50 g water is added to 100 g of the obtained filter cake and the suspension is stirred. Afterwards the suspension is spray-dried a to a powder with a water content of about 13%. 54 g water is added to 46 g of the obtained powder. A thin fluid suspension is obtained.

**Example 2**

The synthesis of Example 1 is repeated. With the exception that the obtained filter cake is dried in vacuum at about 70°C to a water content of about 18.7%.

**Example 3**

The synthesis of Example 1 is repeated. With the exception that the obtained filter cake is dried by a infra-red dryer to a water content of about 13.8%.

**Example 4**

The synthesis of Example 1 is repeated. With the exception that the obtained filter cake is dried by a infra-red

dryer to water content of about 5.2 %.

**Example 5**

A solution consisting of 400 ml water, 45 g of 4,4'-diaminostilbene-2,2'-disulfonic in its sodium salt form and 11.5 g sodium carbonate is added to a mixture consisting of 260 g of methylethylketone, 225 g of ice and 45 g of cyanurchloride. During the addition, the reaction temperature is kept below 10°C by external cooling and the pH is kept between 4.5 and 5 by the addition of 3 g sodium carbonate in 20 ml water. Afterwards 28.6 ml of a 25% ammonia solution are added and the pH value is adjusted between 9.0 and 9.2 by the addition of 6.8 g sodium hydroxide in 35 g water. Afterwards, the reaction solution is stirred for 1 h at 45°C. Then 23 g morpholine is added and the reaction solution is heated up to 70°C. Simultaneously, the pH is adjusted to a value between 8.8 and 9.2 by the addition of 12.5 g sodiumhydroxyd in 70 g water. The reaction solution is stirred for 2 h at 70°C.
Then HCl (32%) is added at 70°C until a pH of 4 is obtained. Afterwards the reaction mixture is heated up to 98°C whereby 320 ml of a mixture of methylethylketone and water are distilled off. The remaining reaction mixture is cooled to 20°C, stirred for 4 h and the precipitation is filtered off and purified by ultrafiltration..

Brief description of the drawings:

Fig. 1. shows the powder X-ray diffraction pattern of the compound of formula (1) obtained from Example 1. The new crystal modification has been analysed by X-ray diffraction on a powder sample of the new crystal modification in the instrument X'Pert powder diffractometer PANalytical inside a closed Anton Paar measuring stage using Cuba radiation. The experimental conditions are mentioned above.

Fig. 2. shows the powder X-ray diffraction pattern of the compound of formula (1) obtained from Example 2. The new crystal modification has been analysed by X-ray diffraction on a powder sample of the new crystal modification in the instrument X'Pert powder diffractometer PANalytical inside a closed Anton Paar measuring stage using $CuK_\alpha$ radiation. The experimental conditions are mentioned above.

Fig. 3. shows the powder X-ray diffraction pattern of the compound of formula (1) obtained from Example 3. The new crystal modification has been analysed by X-ray diffraction on a powder sample of the new crystal modification in the instrument X'Pert powder diffractometer PANalytical inside a closed Anton Paar measuring stage using $CuK_\alpha$ radiation. The experimental conditions are mentioned above.

Fig. 4. shows the powder X-ray diffraction pattern of the compound of formula (1) obtained from Example 4. The new crystal modification has been analysed by X-ray diffraction on a powder sample of the new crystal modification in the instrument X'Pert powder diffractometer PANalytical inside a closed Anton Paar measuring stage using $CuK_\alpha$ radiation. The experimental conditions are mentioned above.

Fig. 5. shows the powder X-ray diffraction pattern of the compound of formula (1') obtained from Example 5. The new crystal modification has been analysed by X-ray diffraction on a powder sample of the new crystal modification in the instrument X'Pert powder diffractometer PANalytical inside a closed Anton Paar measuring stage using $CuK_\alpha$ radiation. The experimental conditions are mentioned above.

**Claims**

**1.** A crystal modification of the compound of formula (1)

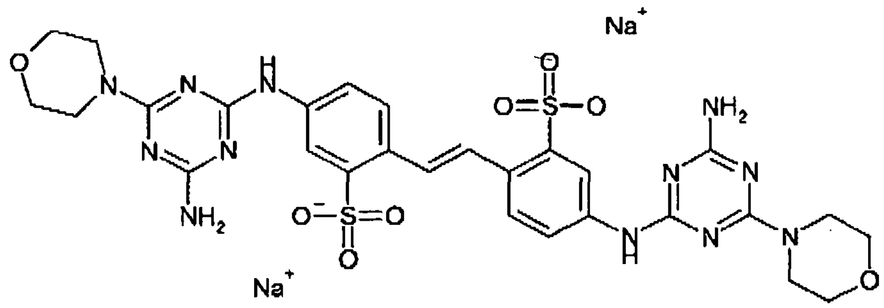

(1)

,

**characterized by**

peaks at about 21.8, 11 -0 and 7.3 d-spacing units in its powder X-ray diffraction pattern at a content of water > 0%.

2. A crystal modification of the compound of formula (1) according to claim 1 with a content of water ≥ 19% **characterized by** peaks at about 21.8, 11.6, 11.0, 9.2, 7.3, 5.82, 4.37, 4.05, 3.96, 3.88, 3.36. 3.1, 3.04, 2.95, 2.8, 2.56 and 2.32 d-spacing units in its powder X-ray diffraction pattern.

3. A crystal modification of the compound of formula (1) according to claim 1 with a content of water < 19% and ≥ 17% **characterized by** peaks at about about 21.8, 11.7, 11.0, 9.2, 7.30, 5.78 and 3.87 d-spacing units in its powder X-ray diffraction pattern.

4. A crystal modification of the compound of formula (1) according to claim 1 with a content of water < 17% and ≥ 13%, **characterized by** peaks at about about 21.8, 11.7, 11.0, 7.30, 5.77, 5.51, 4.93 and 3.65 d-spacing units in its powder X-ray diffraction pattern.

5. A crystal modification of the compound of formula (1) according to claim 1 with a content of water < 13% and > 0%, **characterized by** peaks at about about 21.8, 11.0. 9.0, and 7.3 d-spacing units in its powder X-ray diffraction pattern.

6. A crystal modification of the compound of formula (1')

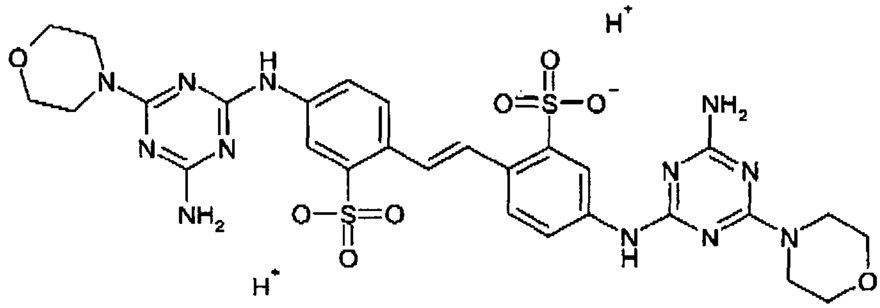

(1')

,

**characterized by**

peaks at about 10.1, 5.62, 3.55 and 3.37 d-spacing units in its powder X-ray diffraction pattern at a content of water > 0%.

7. A crystal modification of the compound of formula (1') according to claim 6 **characterized by** peaks at about 10.1, 5.92, 5.62, 4.21, 3.72, 3.55 and 3.37 d-spacing units in its powder X-ray diffraction pattern.

8. A crystal modification of the mixture comprising a compound of formula (1)

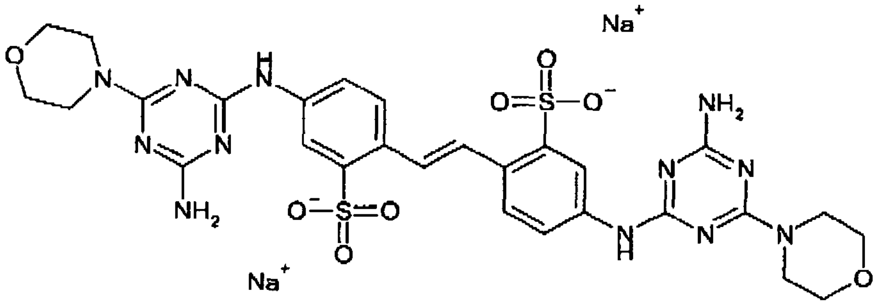

(1)

and

compound of formula (1 a)

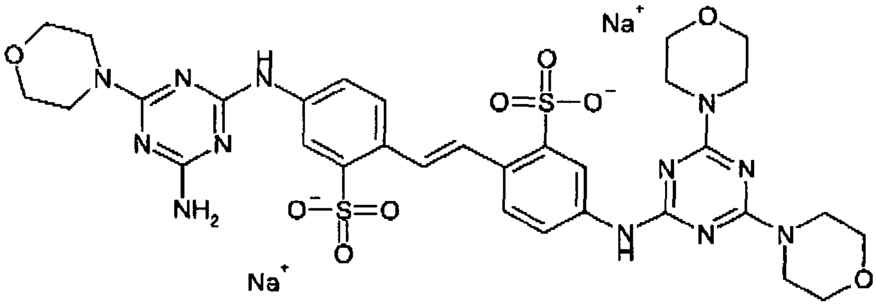

(1a)

**characterized by**

peaks at about 21.8, 11.0 and 7.3 d-spacing units in its powder X-ray diffraction pattern at a content of water > 0%.

**9.** A crystal modification of the mixture according to claim 8 with a content of water ≥ 19% **characterized by** peaks at about 21.8. 11.8, 11.0, 9.2, 7.3, 5.82, 4.48, 4.37, 4.05, 3,95, 3-88, 3.36, 2.95 and 2.56 d-spacing units in its powder X-ray diffraction pattern.

**10.** A crystal modification of the mixture according to claim 8 with a content of water < 19% and ≥ 17% **characterized by** peaks at about 21.8,11.7, 11.5. 11.0, 9.2, 7.3, 7.2, 7.0, 5.78, 5-56, 4.95, 4.75, 4.49, 4.04, 3.87 and 3.71 d-spacing units in its powder X-ray diffraction pattern.

**11.** A crystal modification of the mixture according to claim 8 with a content of water < 17% and s 13% **characterized by** peaks at about 21.8, 11.6, 11.0, 9.2, 7.3, 7.2. 5.77, 5.51, 4.93, 3.86 and 3.65 d-spacing units in its powder X-ray diffraction pattern.

**12.** A crystal modification of the mixture according to claim 8 with a content of water < 13% and ≥ 0% **characterized by** peaks at about 21.8,11.5, 11.0, 9.0, 7.3 and 3.58 d-spacing units in its powder X-ray diffraction pattern.

**13.** A mixture according to claims 8-12, **characterized by** a molar ratio of compound (1) to compound (1a) in the range of 99.99:0.01 to 10:90.

**14.** A mixture according to claim 8 -12, **characterized by** a molar ratio of compound (1) to compound (1a) in the range of 99.99:0.01 to 90:10.

**15.** A crystal modification of the mixture comprising a compound of formula (1')

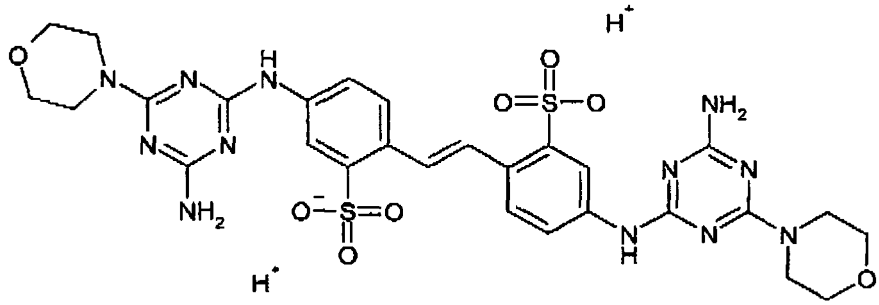

(1')

and
a compound of formula (1'a)

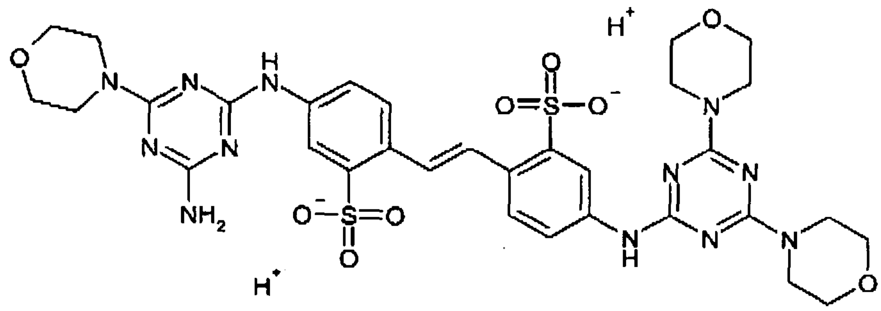

(1'a)

**characterized by**
peaks at about 10.9, 5.62, 3.55 and 3.37 d-spacing units in its powder X-ray diffraction pattern at a content of water > 0%.

**16.** A crystal modification of the mixture according to claim 15 **characterized by** peaks at about 10.1, 5-92, 5.62, 5.05, 4.77, 4.38, 4.21, 9.97, 3.89, 3.72, 3.55, 3-37, 3.06, 2.76, 2.59 and 2.4 d-spacing units in its powder X-ray diffraction pattern.

**17.** A mixture according to claims 15-16. **characterized by** a molar ratio of compound (1') to compound (1'a) in the range of 99.99:0.01 to 10:90.

**18.** A mixture according to claim 15-16, **characterized by** a molar ratio of compound (1') to compound (1'a) in the range of 99.99:0.01 to 90:10.17.

**19.** A mixture comprising

(a) the crystal modification of the compound of formula (1) as defined in Claims 1-5 and/or the crystal modification of the mixture according to claim 8 - 14 and

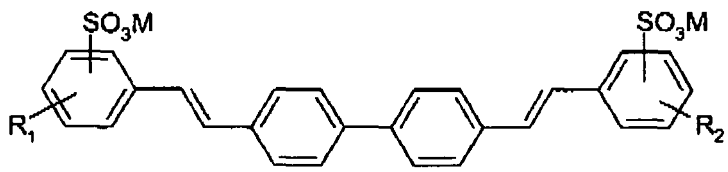

(2),

wherein
$R_1$ and $R_2$, independently of each other, are hydrogen, $C_1$-$C_8$alkyl, $C_1$-$C_8$alkoxy or halogen, and M is hydrogen or a cation.

**20.** A mixture comprising

(b) the crystal modification of the compound of formula (1') as defined in Claims 6 - 7 and/or the crystal modification of the mixture according to claim 15 - 18 and

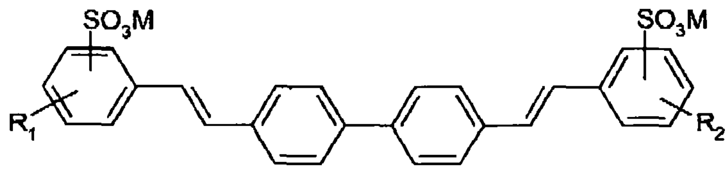

(2),

wherein
$R_1$ and $R_2$, independently of each other, are hydrogen, $C_1$-$C_8$alkyl, $C_1$-$C_8$alkoxy or halogen, and M is hydrogen or a cation.

**21.** A mixture according to Claims 19 and 20, wherein
$R_1$ and $R_2$ are hydrogen and
M is hydrogen, an alkaline- or alkaline earth-metal, or ammonium.

**22.** Use of the crystal modification of the compound of formula (1) according to Claim 1 - 5 as fluorescent whitening agent.

**23.** Use of the crystal modification of the compound of formula (1') according to Claim 6 - 7 as fluorescent whitening agent.

**24.** Use of the crystal modification of the mixture according to claim 8 - 14 as fluorescent whitening agent.

**25.** Use of the crystal modification of the mixture according to claim 15 - 18 as fluorescent whitening agent.

**26.** Use of the mixture according to Claim 19 - 21 as fluorescent whitening agent.

**27.** A detergent composition comprising the crystal modification of the compound of formula (1) according to Claim 1 - 5.

**28.** A detergent composition comprising the crystal modification of the compound of formula (1) according to Claim 6-7.

**29.** A detergent composition comprising the crystal modification of the mixture according to claim 8 - 14.

**30.** A detergent composition comprising the crystal modification of the mixture according to claim 15-18.

**31.** A detergent composition comprising the mixture according to Claim 19 - 21.

**32.** A detergent composition comprising

i) 1 - 70% of an anionic surfactant and/or a nonionic surfactant;
ii) 0 - 75% of a builder;
iii) 0 - 30% of a peroxide;
iv) 0 -10% of a peroxide activator; and
v) 0.001 - 5% of the crystal modification of the compound of formula (1) according to Claim 1 - 5 or comprising the crystal modification of the mixture according to claim 8 - 14 or a mixture according to Claim 19 - 21,
each by weight, based on the total weight of the detergent composition.

**33.** A detergent composition according comprising

i) 1 - 70% of an anionic surfactant and/or a nonionic surfactant;
ii) 0 - 75% of a builder;
iii) 0 - 30% of a peroxide;
iv) 0 -10% of a peroxide activator; and
v) 0.001 - 5% of the crystal modification of the compound of formula (1) according to Claim 6 - 7 or comprising the crystal modification of the mixture according to claim 15 -18, each by weight, based on the total weight of the detergent composition.

**34.** A detergent composition according to claims 32 and 33 comprising

vi) 0.05 - 5% of at least one enzyme selected from the group consisting of cellulase, protease, amylase and lipase, especially protease.

**35.** A method of producing the new crystal modification of the compound of formula (1) as defined in claim 1 - 5 or of the mixture of the compound of formula (1) and the compound of formula (1a) as defined in Claims 8 - 14, **characterized by** reacting in a first step 4,4'-diaminostilbene-2,2'-disulfonic in its sodium salt form, sodium carbonate, methylethylketone and cyanurchloride at low temperature at a pH of between 4 and 5; in a second step morpholino is added and the pH is increased to 8-5 to 9.5 and the temperature is increase to higher temperature (60°C - 110°C) and in a third step the reaction solution is cooled down to lower temperature (10 - 15°C) and the obtained precipitation of the crystal modification of formula (1) or of the crystal modification of the mixture of the compound of formula (1) and the compound of formula (1a) is filtered off and washed by conventional processes.

**36.** A method of producing the new crystal modification of the compound of formula (1') as defined in claim 6 - 7 or of the mixture of the compound of formula (1') and the compound of formula (1'a) as defined in Claims 15 -18, **characterized by** reacting in a first step 4,4'-diaminostilbene-2,2'-disulfonic in its sodium salt form, sodium carbonate, methylethylketone and cyanurchloride at low temperature at a pH of between 4 and 5; in a second step morpholino is added and the pH is increased to 8.5 to 9.5 and the temperature is increase to higher temperature (60°C -110°C), whereby an acid, such as HCl is added until a pH of 4 is reached and the reaction solution is cooled down to lower temperature (10-15°C) and the obtained precipitation of the crystal modification of formula (1') or of the new crystal modification of the mixture of the compound of formula (1') and the compound of formula (1'a) is filtered off and washed by conventional processes.

## Patentansprüche

**1.** Kristallmodifikation der Verbindung der Formel (1)

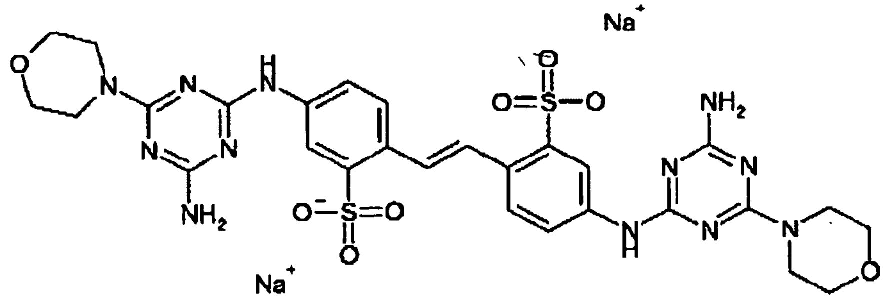

(1)

**dadurch gekennzeichnet, dass**
sie Peaks bei ungefähr 21,8, 11,0 und 7,3 d-Abstandseinheiten in ihrem Röntgenpulverbeugungsmuster bei einem Wassergehalt > 0 % zeigt.

**2.** Kristallmodifikation der Verbindung der Formel (1) nach Anspruch 1 mit einem Wassergehalt ≥ 19 %, **dadurch gekennzeichnet, dass** sie Peaks bei ungefähr 21,8, 11,6, 11,0, 9,2, 7,3, 5,82, 4,37, 4,05, 3,96, 3,88, 3,36, 3,1, 3,04, 2,95, 2,8, 2,56 und 2,32 d-Abstandseinheiten in ihrem Röntgenpulverbeugungsmuster zeigt.

**3.** Kristallmodifikation der Verbindung der Formel (1) nach Anspruch 1 mit einem Wassergehalt < 19 % und ≥ 17 %, **dadurch gekennzeichnet, dass** sie Peaks bei ungefähr 21,8, 11,7, 11,0, 9,2, 7,30, 5,78 und 3,87 d-Abstandseinheiten in ihrem Röntgenpulverbeugungsmuster zeigt.

**4.** Kristallmodifikation der Verbindung der Formel (1) nach Anspruch 1 mit einem Wassergehalt < 17 % und ≥ 13 %, **dadurch gekennzeichnet, dass** sie Peaks bei ungefähr 21,8, 11,7, 11,0, 7,30, 5,77, 5,51, 4,93 und 3,65 d-Abstandseinheiten in ihrem Röntgenpulverbeugungsmuster zeigt.

**5.** Kristallmodifikation der Verbindung der Formel (1) nach Anspruch 1 mit einem Wassergehalt < 13 % und > 0 %,

**dadurch gekennzeichnet, dass** sie Peaks bei ungefähr 21,8, 11,0, 9,0 und 7,3 d-Abstandseinheiten in ihrem Röntgenpulverbeugungsmuster zeigt.

6. Kristallmodifikation der Verbindung der Formel (1')

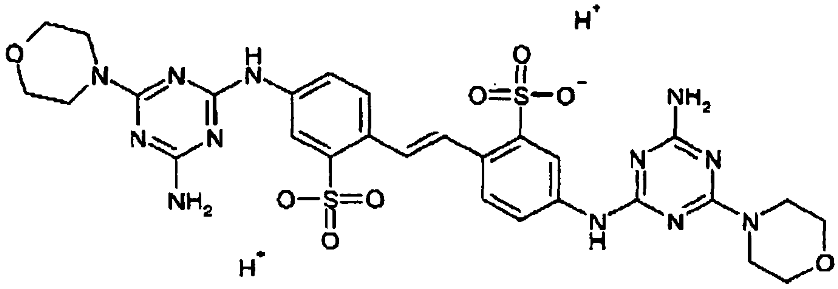

(1')

**dadurch gekennzeichnet, dass**
sie Peaks bei ungefähr 10,1, 5,62, 3,55 und 3,37 d-Abstandseinheiten in ihrem Röntgenpulverbeugungsmuster bei einem Wassergehalt > 0 % zeigt.

7. Kristallmodifikation der Verbindung der Formel (1') nach Anspruch 6, **dadurch gekennzeichnet, dass** sie Peaks bei ungefähr 10,1, 5,92, 5,62, 4,21, 3,72, 3,55 und 3,37 d-Abstandseinheiten in ihrem Röntgenpulverbeugungsmuster zeigt.

8. Kristallmodifikation der Mischung, umfassend eine Verbindung der Formel (1)

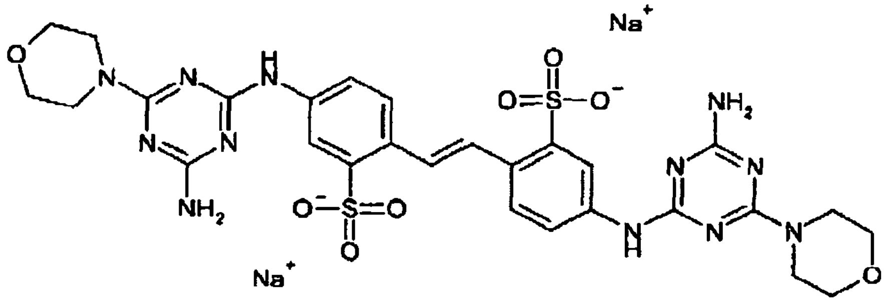

(1)

und
eine Verbindung der Formel (1a)

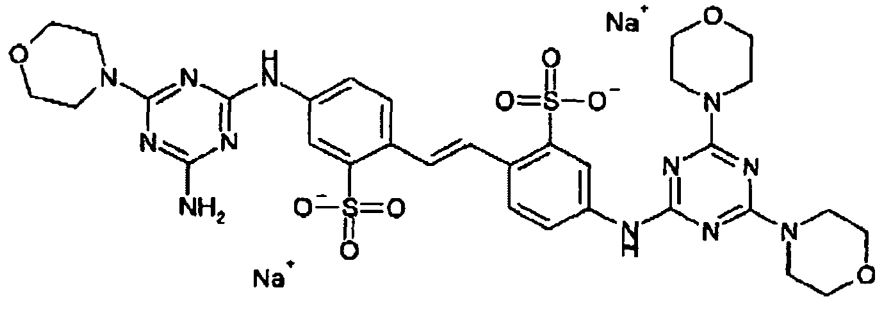

(1a)

**dadurch gekennzeichnet, dass**
sie Peaks bei ungefähr 21,8, 11,0 und 7,3 d-Abstandseinheiten in ihrem Röntgenpulverbeugungsmuster bei einem Wassergehalt > 0 % zeigt.

**9.** Kristallmodifikation der Mischung nach Anspruch 8 mit einem Wassergehalt ≥ 19 %, **dadurch gekennzeichnet, dass** sie Peaks bei ungefähr 21,8, 11,8, 11,0, 9,2, 7,3, 5,82, 4,48, 4,37, 4,05, 3,96, 3,88, 3,36, 2,95 und 2,56 d-Abstandseinheiten in ihrem Röntgenpulverbeugungsmuster zeigt.

**10.** Kristallmodifikation der Mischung nach Anspruch 8 mit einem Wassergehalt < 19 % und ≥ 17 %, **dadurch gekennzeichnet, dass** sie Peaks bei ungefähr 21,8, 11,7, 11,5, 11,0, 9,2, 7,3, 7,2, 7,0, 5,78, 5,56, 4,95, 4,75, 4,49, 4,04, 3,87 und 3,71 d-Abstandseinheiten in ihrem Röntgenpulverbeugungsmuster zeigt.

**11.** Kristallmodifikation der Mischung nach Anspruch 8 mit einem Wassergehalt < 17 % und ≥ 13 %, **dadurch gekennzeichnet, dass** sie Peaks bei ungefähr 21,8, 11,6, 11,0, 9,2, 7,3, 7,2, 5,77, 5,51, 4,93, 3,86 und 3,65 d-Abstandseinheiten in ihrem Röntgenpulverbeugungsmuster zeigt.

**12.** Kristallmodifikation der Mischung nach Anspruch 8 mit einem Wassergehalt < 13 % und ≥ 0 %, **dadurch gekennzeichnet, dass** sie Peaks bei ungefähr 21,8, 11,5, 11,0, 9,0, 7,3 und 3,58 d-Abstandseinheiten in ihrem Röntgenpulverbeugungsmuster zeigt.

**13.** Mischung nach einem der Ansprüche 8 - 12, **dadurch gekennzeichnet, dass** das Molverhältnis der Verbindung (1) zu der Verbindung (1a) in dem Bereich von 99,99 : 0,01 bis 10 : 90 liegt.

**14.** Mischung nach einem der Ansprüche 8 - 12, **dadurch gekennzeichnet, dass** das Molverhältnis der Verbindung (1) zu der Verbindung (1a) in dem Bereich von 99,99 : 0,01 bis 90 : 10 liegt.

**15.** Kristallmodifikation der Mischung umfassend eine Verbindung der Formel (1')

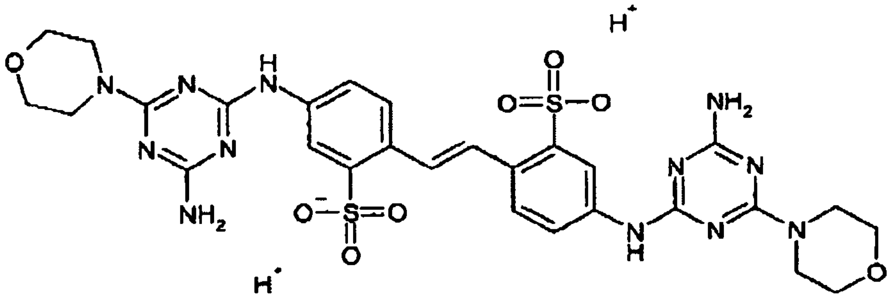

(1')

und
eine Verbindung der Formel (1'a)

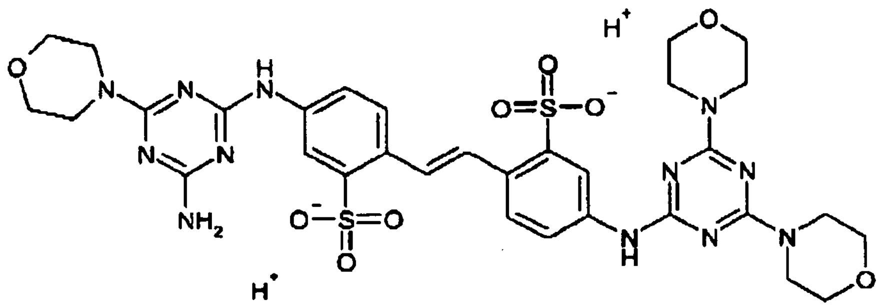

(1'a)

**dadurch gekennzeichnet, dass**
sie Peaks bei ungefähr 10,1, 5,62, 3,55 und 3,37 d-Abstandseinheiten in ihrem Röntgenpulverbeugungsmuster bei einem Wassergehalt > 0 % zeigt.

**16.** Kristallmodifikation der Mischung nach Anspruch 15, **dadurch gekennzeichnet, dass** sie Peaks bei ungefähr 10,1, 5,92, 5,62, 5,05, 4,77, 4,38, 4,21, 9,97, 3,89, 3,72, 3,55, 3,37, 3,06, 2,76, 2,59 und 2,4 d-Abstandseinheiten in ihrem Röntgenpulverbeugungsmuster zeigt.

**17.** Mischung nach einem der Ansprüche 15 - 16, **dadurch gekennzeichnet, dass** das Molverhältnis der Verbindung (1') zu der Verbindung (1'a) in dem Bereich von 99,99 : 0,01 bis 10 : 90 liegt.

**18.** Mischung nach einem der Ansprüche 15 - 16, **dadurch gekennzeichnet, dass** das Molverhätnis der Verbindung (1') zu der Verbindung (1'a) in dem Bereich von 99,99 : 0,01 bis 90 : 10,17 liegt.

**19.** Mischung, umfassend

(a) die Kristallmodifikation der Verbindung der Formel (1) nach einem der Ansprüche 1 - 5 und/oder die Kristallmodifikation der Mischung nach einem der Ansprüche 8 - 14 und

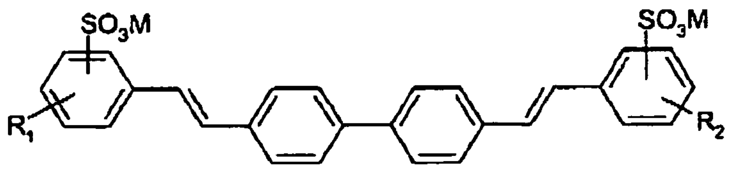

(2)

worin
$R_1$ und $R_2$ unabhängig voneinander Wasserstoff, $C_1$-$C_8$-Alkyl, $C_1$-$C_8$-Alkoxy oder Halogen sind und M Wasserstoff oder ein Kation darstellt.

**20.** Mischung, umfassend

(b) die Kristallmodifikation der Verbindung der Formel (1') nach einem der Ansprüche 6 - 7 und/oder die Kristallmodifikation der Mischung nach einem der Ansprüche 15 - 18 und

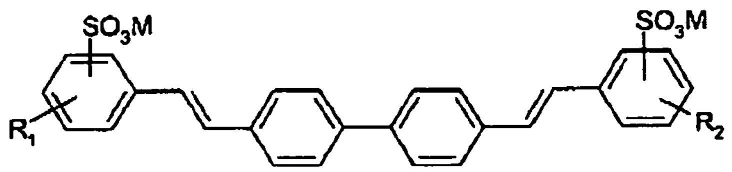

(2)

worin
$R_1$ und $R_2$ unabhängig voneinander Wasserstoff, $C_1$-$C_8$-Alkyl, $C_1$-$C_8$-Alkoxy oder Halogen sind und M Wasserstoff oder ein Kation darstellt.

**21.** Mischung nach Anspruch 19 und 20, worin
$R_1$ und $R_2$ Wasserstoff sind und
M Wasserstoff, ein Alkalimetall oder ein Erdalkalimetall oder Ammonium ist.

**22.** Verwendung der Kristallmodifikation der Verbindung der Formel (1) nach einem der Ansprüche 1 - 5 als fluoreszierenden Weißmacher.

**23.** Verwendung der Kristallmodifikation der Verbindung der Formel (1') nach einem der Ansprüche 6 - 7 als fluoreszierenden Weißmacher.

**24.** Verwendung der Kristallmodifikation der Mischung nach einem der Ansprüche 8 - 14 als fluoreszierenden Weißmacher.

**25.** Verwendung der Kristallmodifikation der Mischung nach einem der Ansprüche 15 - 18 als fluoreszierenden Weißmacher.

**26.** Verwendung der Mischung nach einem der Ansprüche 19 - 21 als fluoreszierenden Weißmacher.

**27.** Detergenszusammensetzung, umfassend die Kristallmodifikation der Verbindung der Formel (1) nach einem der Ansprüche 1 - 5.

**28.** Detergenszusammensetzung, umfassend die Kristallmodifikation der Verbindung der Formel (1) nach einem der Ansprüche 6 - 7.

**29.** Detergenszusammensetzung, umfassend die Kristallmodifikation der Mischung nach einem der Ansprüche 8 - 14.

**30.** Detergenszusammensetzung, umfassend die Kristallmodifikation der Mischung nach einem der Ansprüche 15 - 18.

**31.** Detergenszusammensetzung, umfassend die Mischung nach einem der Ansprüche 19 - 21.

**32.** Detergenszusammensetzung, umfassend

i) 1 - 70 % eines anionischen Tensids und/oder eines nichtionischen Tensids;
ii) 0 - 75 % eines Builders;
iii) 0 - 30 % eines Peroxids;
iv) 0-10 % eines Peroxidaktivators; und
v) 0,001 - 5 % der Kristallmodifikation der Verbindung der Formel (1) nach einem der Ansprüche 1 - 5 oder umfassend die Kristallmodifikation der Mischung nach einem der Ansprüche 8 - 14 oder eine Mischung nach einem der Ansprüche 19-21,
jeweils bezogen auf das Gewicht auf der Basis des Gesamtgewichts der Detergenszusammensetzung.

**33.** Detergenszusammensetzung, umfassend

i) 1 - 70 % eines anionischen Tensids und/oder eines nichtionischen Tensids;
ii) 0 - 75 % eines Builders;
iii) 0 - 30 % eines Peroxids;
iv) 0-10 % eines Peroxidaktivators; und
v) 0,001 - 5 % der Kristallmodifikation der Verbindung der Formel (1) nach einem der Ansprüche 6 - 7 oder umfassend die Kristallmodifikation der Mischung nach einem der Ansprüche 15 - 18,
jeweils bezogen auf das Gewicht auf der Basis des Gesamtgewichts der Detergenszusammensetzung.

**34.** Detergenszusammensetzung nach den Ansprüchen 32 und 33, umfassend

vi) 0,05 - 5 % mindestens eines Enzyms, ausgewählt aus der Gruppe bestehend aus Cellulase, Protease, Amylase und Lipase, insbesondere Protease.

**35.** Verfahren zur Herstellung der neuen Kristallmodifikation der Verbindung der Formel (1), wie sie in einem der Ansprüche 1 - 5 definiert ist, oder der Mischung der Verbindung der Formel (1) und der Verbindung der Formel (1a), wie sie in einem der Ansprüche 8 - 14 definiert ist, **dadurch gekennzeichnet, dass** in einem ersten Schritt 4,4'-Diaminostilben-2,2'-disulfonsäure in ihrer Natriumsalzform, Natriumcarbonat, Methylethylketon und Cyanurchlorid bei niedriger Temperatur bei einem pH-Wert von zwischen 4 und 5 umgesetzt werden; in einem zweiten Schritt Morpholin zugesetzt wird und der pH-Wert auf 8,5 bis 9,5 erhöht wird und die Temperatur auf eine höhere Temperatur (60°C - 110 °C) erhöht wird und in einem dritten Schritt die Reaktionslösung auf eine niedrigere Temperatur (10 - 15°C) abgekühlt wird und der erhaltene Niederschlag der Kristallmodifikation der Formel (1) oder der Kristallmodifikation der Mischung der Verbindung der Formel (1) und der Verbindung der Formel (1a) durch herkömmliche Verfahren abfiltriert und gewaschen wird.

**36.** Verfahren zur Herstellung der neuen Kristallmodifikation der Verbindung der Formel (1'), wie sie in einem der Ansprüche 6 - 7 definiert ist, oder der Mischung der Verbindung der Formel (1') und der Verbindung der Formel (1'a), wie sie in einem der Ansprüche 15 - 18 definiert ist, **dadurch gekennzeichnet, dass** in einem ersten Schritt 4,4'-Diaminostilben-2,2'-disulfonsäure in ihrer Natriumsalzform, Natriumcarbonat, Methylethylketon und Cyanurchlorid bei niedriger Temperatur bei einem pH-Wert von zwischen 4 und 5 umgesetzt werden; in einem zweiten Schritt Morpholin zugesetzt wird und der pH-Wert auf 8,5 bis 9,5 erhöht wird und die Temperatur auf eine höhere Temperatur (60°C - 110 °C) erhöht wird, wobei eine Säure, wie HCl, so lange zugesetzt wird, bis ein pH-Wert von 4 erreicht wird, und die Reaktionslösung auf eine niedrigere Temperatur (10 - 15 °C) abgekühlt wird und der erhaltene Niederschlag der Kristallmodifikation der Formel (1') oder der neuen Kristallmodifikation der Mischung der Verbindung der Formel (1') und der Verbindung der Formel (1'a) durch herkömmliche Verfahren abfiltriert und gewaschen wird.

**Revendications**

1. Modification de cristal du composé de formule (1)

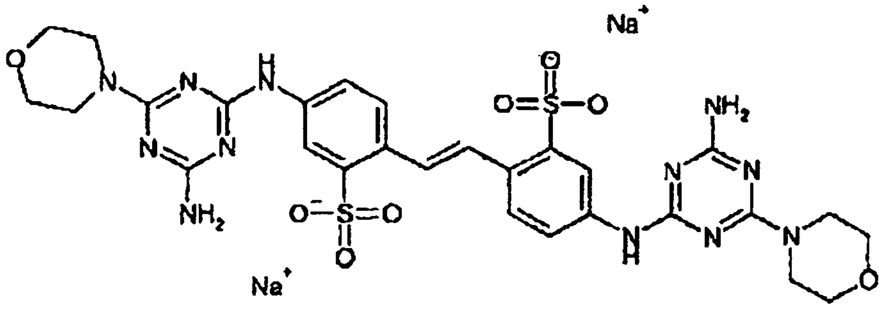

(1)

**caractérisée par**
des pics d'espacement d à environ 21,8, 11,0 et 7,3 dans son motif de diffraction aux rayons X sur poudre à une teneur en eau > 0 %.

2. Modification de cristal du composé de formule (1) selon la revendication 1, ayant une teneur en eau ≥ 19 % **caractérisée par** des pics d'espacement d à environ 21,8, 11,6, 11,0, 9,2, 7,3, 5,82, 4,37, 4,05, 3,96, 3,88, 3,36, 3,1, 3,04, 2,95, 2,8, 2,56 et 2,32 dans son motif de diffraction aux rayons X sur poudre.

3. Modification de cristal du composé de formule (1) selon la revendication 1, ayant une teneur en eau < 19 % et ≥ 17 % **caractérisée par** des pics d'espacement d à environ 21,8, 11,7, 11,0, 9,2, 7,30, 5,78 et 3,87 dans son motif de diffraction aux rayons X sur poudre.

4. Modification de cristal du composé de formule (1) selon la revendication 1, ayant une teneur en eau < 17 % et 13 % **caractérisée par** des pics d'espacement d à environ 21,8, 11,7, 11,0, 7,30, 5,77, 5, 51, 4,93 et 3,65 dans son motif de diffraction aux rayons X sur poudre.

5. Modification de cristal du composé de formule (1) selon la revendication 1, ayant une teneur en eau < 13 % et > 0 % **caractérisée par** des pics d'espacement d à environ 21,8, 11,0, 9,0 et 7,3 dans son motif de diffraction aux rayons X sur poudre.

6. Modification de cristal du composé de formule (1')

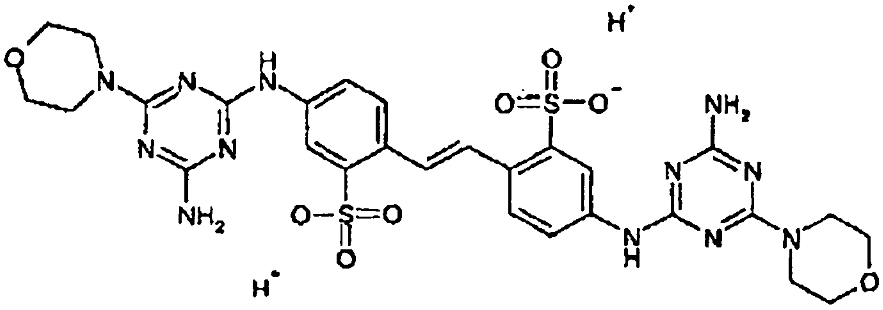

(1')

**caractérisée par**
des pics d'espacement d à environ 10,1, 5,62, 3,55 et 3,37 dans son motif de diffraction aux rayons X sur poudre à une teneur en eau > 0 %.

7. Modification de cristal du composé de formule (1') selon la revendication 6, **caractérisée par** des pics d'espacement d à environ 10,1, 5,92, 5,62, 4,21, 3,72, 3,55 et 3,37 dans son motif de diffraction aux rayons X sur poudre.

8. Modification de cristal du mélange comprenant un composé de formule (1)

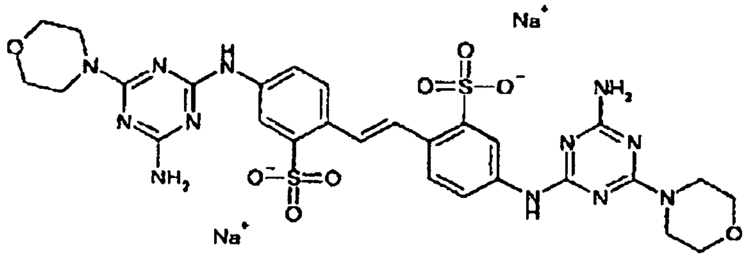

(1)

et

composé de formule (1a)

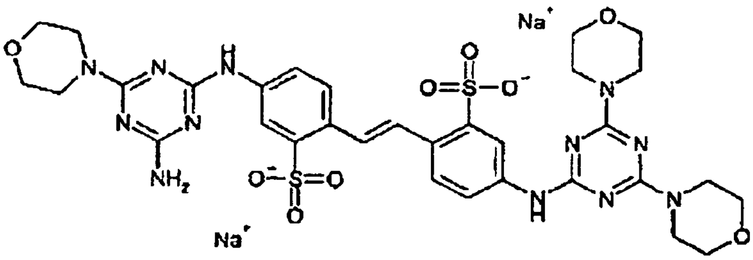

(1a)

**caractérisée par**

des pics d'espacement d à environ 21,8, 11,0 et 7,3 dans son motif de diffraction aux rayons X sur poudre à une teneur en eau > 0 %.

9. Modification de cristal du mélange selon la revendication 8, ayant une teneur en eau ≥ 19 % **caractérisée par** des pics d'espacement d à environ 21,8, 11,8, 11,0, 9,2, 7,3, 5,82, 4,48, 4,37, 4,05, 3,96, 3,88, 3,36, 2,95 et 2,56 dans son motif de diffraction aux rayons X sur poudre.

10. Modification de cristal du mélange selon la revendication 8, ayant une teneur en eau < 19 % et ≥ 17 % **caractérisée par** des pics d'espacement d à environ 21,8, 11,7, 11,5, 11,0, 9,2, 7,3, 7,2, 7,0, 5,78, 5,56, 4,95, 4,75, 4,49, 4,04, 3,87 et 3,71 dans son motif de diffraction aux rayons X sur poudre.

11. Modification de cristal du mélange selon la revendication 8, ayant une teneur en eau < 17 % et ≥ 13 % **caractérisée par** des pics d'espacement d à environ 21,8, 11,6, 11,0, 9,2, 7,3, 7,2, 5,77, 5,51, 4,93, 3,86 et 3,65 dans son motif de diffraction aux rayons X sur poudre.

12. Modification de cristal du mélange selon la revendication 8, ayant une teneur en eau < 13 % et > 0 % **caractérisée par** des pics d'espacement d à environ 21,8, 11,5, 11,0, 9,0, 7,3 et 3,58 dans son motif de diffraction aux rayons X sur poudre.

13. Mélange selon les revendications 8 à 12, **caractérisé par** un rapport molaire de composé (1) sur le composé (1a) dans la gamme de 99,99 : 0,01 à 10 : 90.

14. Mélange selon les revendications 8 à 12, **caractérisé par** un rapport molaire de composé (1) sur le composé (1a) dans la gamme de 99,99 : 0,01 à 90 : 10.

15. Modification de cristal du mélange comprenant un composé de formule (1')

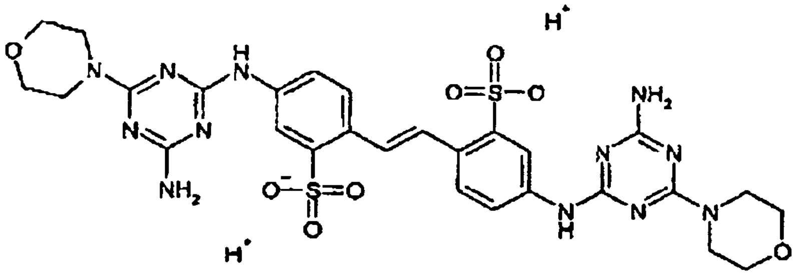

(1')

et
un composé de formule (1'a)

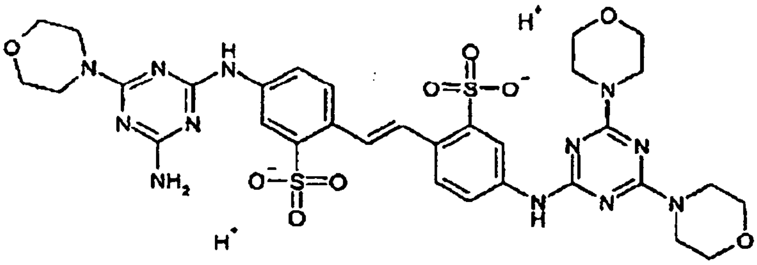

(1'a)

**caractérisée par**
des pics d'espacement d à environ 10,1, 5,62, 3,55 et 3,37 dans son motif de diffraction aux rayons X sur poudre à une teneur en eau > 0 %.

16. Modification de cristal du mélange selon la revendication 15, **caractérisée par** des pics d'espacement d à environ 10,1, 5,92, 5,62, 5,05, 4,77, 4,38, 4,21, 9,97, 3,89, 3,72, 3,55, 3,37, 3,06, 2,76, 2,59 et 2,4 dans son motif de diffraction aux rayons X sur poudre.

17. Un mélange selon les revendications 15 et 16, **caractérisé par** un rapport molaire de composé (1') sur le composé (1'a) dans la gamme de 99,99 : 0,01 à 10 : 90.

18. Un mélange selon les revendications 15 et 16, **caractérisé par** un rapport molaire de composé (1') sur le composé (1'a) dans la gamme de 99,99 : 0,01 à 90: 10,17.

19. Un mélange comprenant

(a) la modification de cristal du composé de formule (1) comme défini dans les revendications 1 à 5 et/ou la modification de cristal du mélange selon les revendications 8 à 14 et

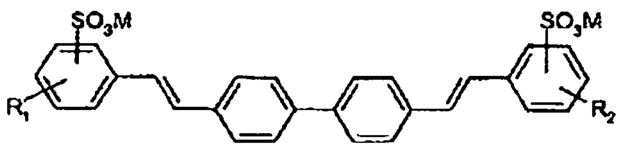

(2)

dans laquelle
$R_1$ et $R_2$, indépendamment l'un de l'autre, sont l'hydrogène, un groupe alkyle en $C_1$ à $C_8$, alcoxy en $C_1$ à $C_8$ ou un halogène, et M est l'hydrogène ou un cation.

20. Un mélange comprenant

(b) la modification de cristal du composé de formule (1') comme défini dans les revendications 6 et 7 et/ou la

modification de cristal du mélange selon les revendications 15 à 18 et

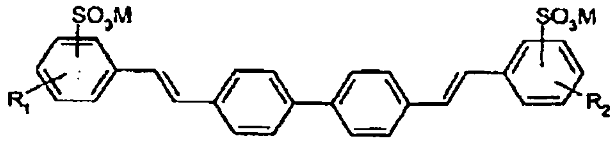 (2)

,

dans laquelle
$R_1$ et $R_2$, indépendamment l'un de l'autre, sont l'hydrogène, un groupe alkyle en $C_1$ à $C_8$, alcoxy en $C_1$ à $C_8$ ou un halogène, et M est l'hydrogène ou un cation.

**21.** Mélange selon les revendications 19 et 20, dans lequel
$R_1$ et $R_2$ sont l'hydrogène et
M est l'hydrogène, un métal alcalin ou alcalino-terreux, ou l'ammonium.

**22.** Utilisation de la modification de cristal du composé de formule (1) selon les revendications 1 à 5 en tant qu'azurant fluorescent.

**23.** Utilisation de la modification de cristal du composé de formule (1') selon les revendications 6 et 7 en tant qu'azurant fluorescent.

**24.** Utilisation de la modification de cristal du mélange selon les revendications 8 à 14 en tant qu'azurant fluorescent.

**25.** Utilisation de la modification de cristal du mélange selon les revendications 15 à 18 en tant qu'azurant fluorescent.

**26.** Utilisation du mélange selon les revendications 19 à 21 en tant qu'azurant fluorescent.

**27.** Une composition détergente comprenant la modification de cristal du composé de formule (1) selon les revendications 1 à 5.

**28.** Une composition détergente comprenant la modification de cristal du composé de formule (1) selon les revendications 6 et 7.

**29.** Une composition détergente comprenant la modification de cristal du mélange selon les revendications 8 à 14.

**30.** Une composition détergente comprenant la modification de cristal du mélange selon les revendications 15 à 18.

**31.** Une composition détergente comprenant le mélange selon les revendications 19 à 21.

**32.** Une composition détergente comprenant

i) 1 à 70 % d'un agent tensioactif anionique et/ou d'un agent tensioactif non ionique ;
ii) 0 à 75 % d'un adjuvant ;
iii) 0 à 30 % d'un peroxyde ;
iv) 0 à 10 % d'un activateur de peroxyde ; et
v) 0,001 à 5 % de la modification de cristal du composé de formule (1) selon les revendications 1 à 5 ou comprenant la modification de cristal du mélange selon les revendications 8 à 14 ou un mélange selon les revendications 19 à 21,
chacun en poids, sur la base du poids total de la composition détergente.

**33.** Composition détergente comprenant

i) 1 à 70 % d'un agent tensioactif anionique et/ou d'un agent tensioactif non ionique ;
ii) 0 à 75 % d'un adjuvant ;
iii) 0 à 30 % d'un peroxyde ;

iv) 0 à 10 % d'un activateur de peroxyde ; et

v) 0,001 à 5 % de la modification de cristal du composé de formule (1) selon les revendications 6 et 7 ou comprenant la modification de cristal du mélange selon les revendications 15 à 18,chacun en poids, sur la base du poids total de la composition détergente.

**34.** Une composition détergente selon les revendications 32 et 33, comprenant

vi) 0,05 à 5 % d'au moins une enzyme choisie dans le groupe constitué par la cellulase, la protéase, l'amylase et la lipase, spécialement la protéase.

**35.** Procédé de production de la nouvelle modification de cristal du composé de formule (1) comme défini dans les revendications 1 à 5 ou du mélange du composé de formule (1) et du composé de formule (1a) comme défini dans les revendications 8 à 14, **caractérisé par** la réaction dans une première étape de l'acide 4,4'-diaminostilbène-2,2'-disulfonique sous sa forme de sel de sodium, carbonate de sodium, méthyléthylcétone et cyanurchlorure à basse température à un pH compris entre 4 et 5 ; dans une deuxième étape, le dérivé morpholino est ajouté et le pH est augmenté de 8,5 à 9,5 et la température est augmentée à une température plus élevée (60 °C à 110 °C) et dans une troisième étape, la solution de réaction est refroidie à une température inférieure (10 à 15°C) et la précipitation obtenue de la modification de cristal de formule (1) ou de la modification de cristal du mélange du composé de formule (1) et du composé de formule (1a) est éliminée par filtration et lavée par des procédés classiques.

**36.** Procédé de production de la nouvelle modification de cristal du composé de formule (1') comme défini dans les revendications 6 et 7 ou du mélange du composé de formule (1') et du composé de formule (1'a) comme défini dans les revendications 15 à 18, **caractérisé par** la réaction dans une première étape de l'acide 4,4'-diaminostilbène-2,2'-disulfonique sous sa forme de sel de sodium, carbonate de sodium, méthyléthylcétone et cyanurchlorure à basse température à un pH compris entre 4 et 5 ; dans une deuxième étape, le dérivé morpholino est ajouté et le pH est augmenté à 8,5 à 9,5 et la température est augmentée à une température plus élevée (60 °C à 110 °C), moyennant quoi un acide, tel que HCl est ajouté jusqu'à atteindre un pH de 4 et la solution de réaction est refroidie à une température inférieure (10 à 15°C) et la précipitation obtenue de la nouvelle modification de cristal de formule (1') ou de la modification de cristal du mélange du composé de formule (1') et du composé de formule (1'a) est éliminée par filtration et lavée par des procédés classiques.

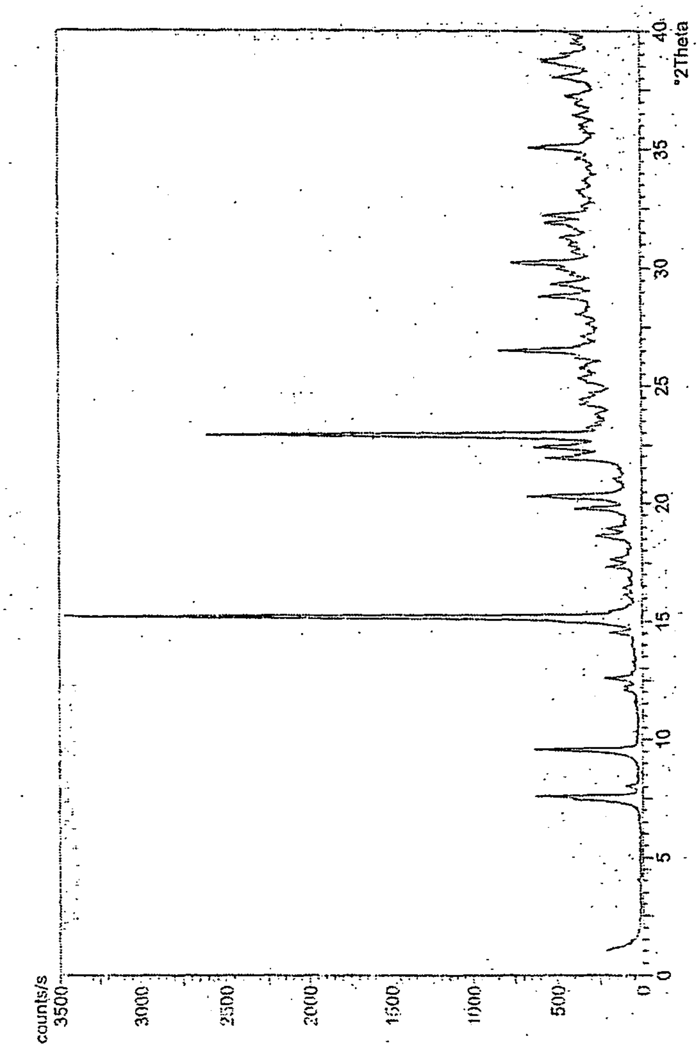


Fig.1: shows the powder X-ray diffraction pattern of the compound of formula (1) obtained from example 1.

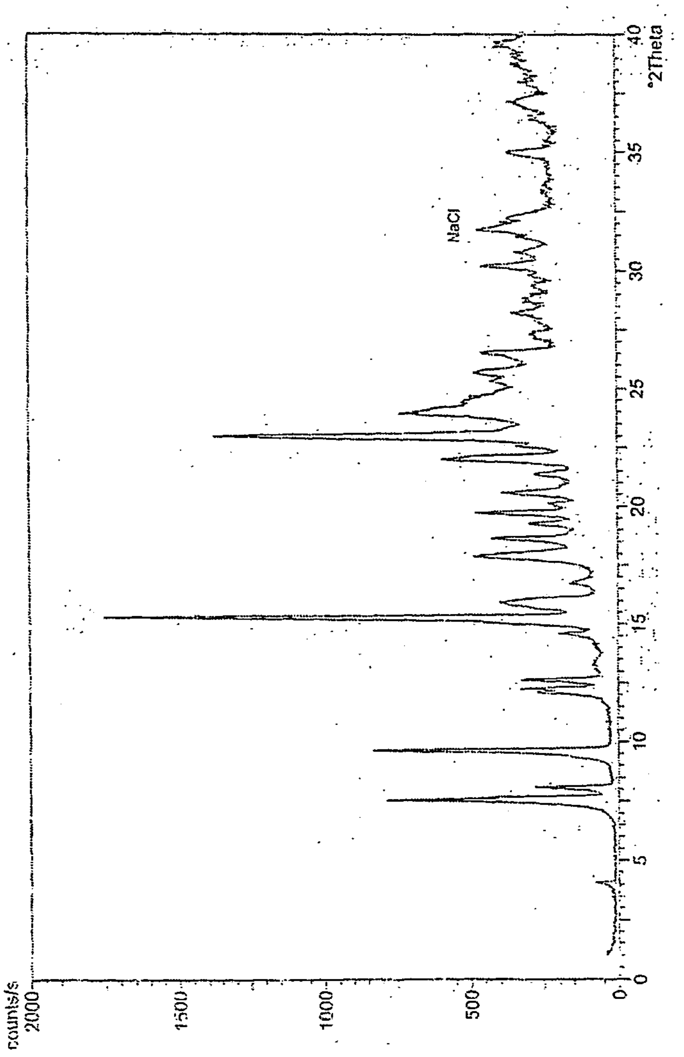


**Fig.2**: shows the powder X-ray diffraction pattern of the compound of formula (1) obtained from example 2.

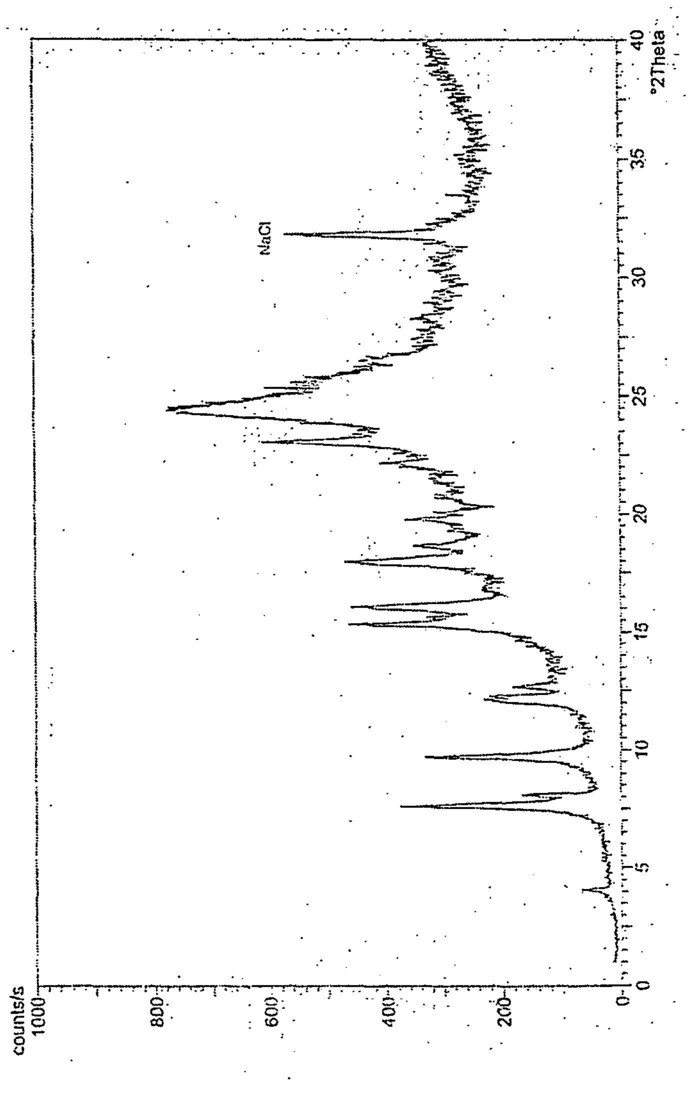


Fig.3: shows the powder X-ray diffraction pattern of the compound of formula (1) obtained from example 3.

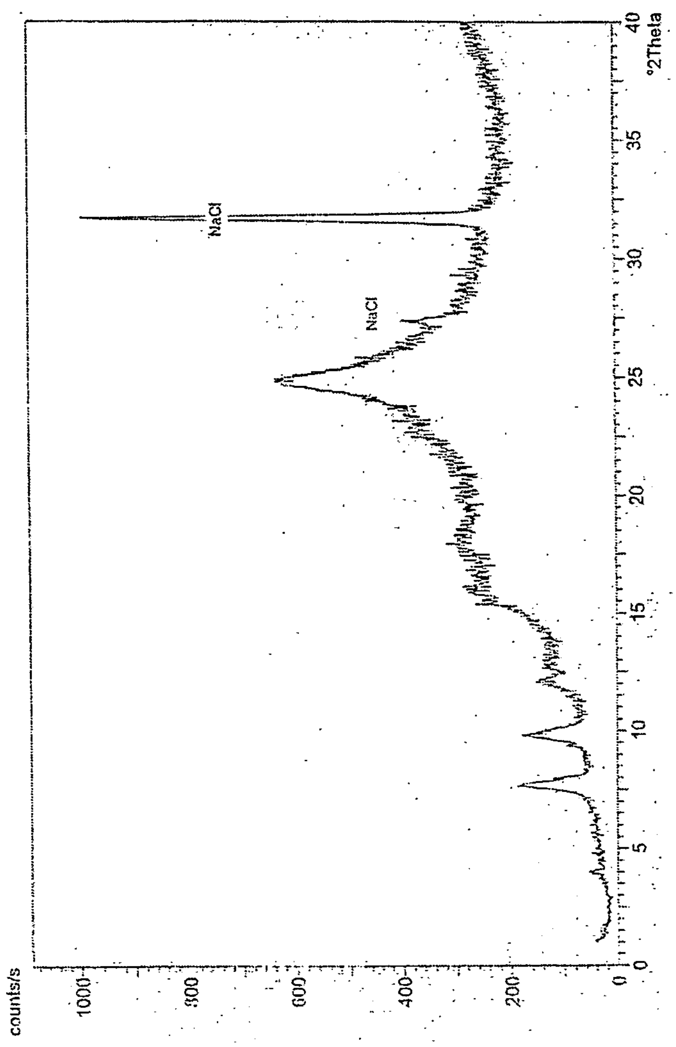


Fig.4: shows the powder X-ray diffraction pattern of the compound of formula (1) obtained from example 4.

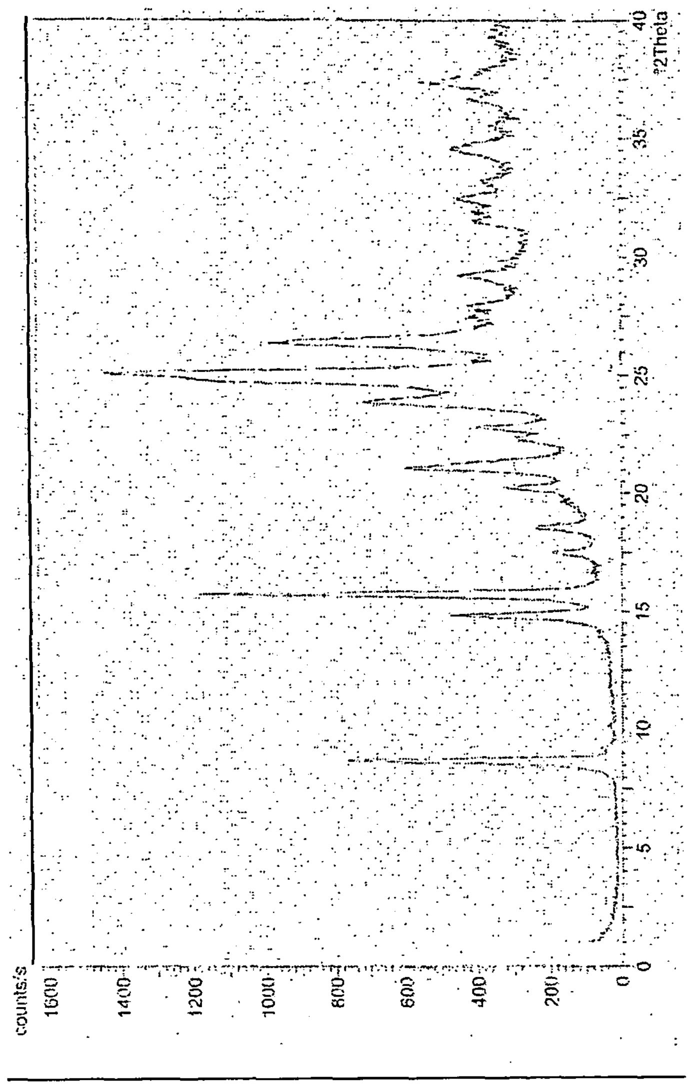


Fig.5: shows the powder X-ray diffraction pattern of the compound of formula (1) obtained from example 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003070870 A
- DD 145017
- US 4005026 A
- GB 1093507 A
- GB 2158454 A
- DE 4443177 A
- EP 0509787 A
- US 6242405 B
- WO 0202865 A